# EUROPEAN PATENT SPECIFICATION

(11) **EP 1 966 367 B1**
(45) Date of publication and mention of the grant of the patent: **03.03.2010**
(21) Application number: 06828762.2
(22) Date of filing: 22.12.2006
(51) Int. Cl.: C12M 3/02, C12M 3/06, G01N 33/50, C12Q 1/68, C12N 5/07, C12M 3/04

(54) **BIOREACTOR FOR CELL AND TISSUE CULTURE**
BIOREAKTOR FÜR DIE ZELL- UND GEWEBEKULTUR
BIORÉACTEUR POUR CULTURES DE CELLULES ET DE TISSUS

(30) Priority: 30.12.2005 DK 200501852; 11.08.2006 US 836943 P
(43) Date of publication of application: 10.09.2008
(73) Proprietor: Drugmode ApS, 5230 Odense M (DK)
(72) Inventor: LARSEN,Peter Mose, DK-5260 Odense (DK); FEY, Stephen John, DK-5491 Blommenslyst (DK)
(74) Representative: Orsnes, Henrik Egede
(86) International application number: PCT/DK2006/000740
(87) International publication number: WO 2007/076865

(56) References cited:
- WO-A-00/00586
- WO-A-01/96866
- WO-A-02/38735
- WO-A-02/42409
- WO-A-97/28252
- US-A- 5 153 131
- US-A- 5 702 941
- US-A1- 2002 146 816
- US-A1- 2004 009 495

## Description

### Field of the invention

The present invention relates to a bioreactor or a culture vessel for incubation of one or more cell cultures, tissue biopsies, cell clusters, tissue-like structures, "prototissues" or similar samples.

### Background of the invention

During "classical" cell culture in an essentially flat culture vessel, cells in general and biopsies in particular tend to de-differentiate. Visibly, biopsies exhibit the 'melting ice-cream effect' as cells migrate from a block of tissue out onto the flat supporting surface of the culture vessel. Gene expression is altered in these "migrating" cells, which begin to behave biochemically as isolated cells rather than as cellular components of a differentiated tissue. De-differentiated cells express different biochemical pathways than those normally expressed by corresponding cells in an intact organism.

In contrast with "classical" cell culture conditions, "microgravity" conditions preserve the differentiation state of many types of cells in culture. Microgravity bioreactors maintain microgravity conditions by continuous rotation of a typically cylindrical or tubular incubation compartment. This rotation continuously forces cells towards the center of the incubation chamber, suspending the cells in a fluid environment using a minimum shear force. This induces them to interact and to aggregate into colonies. For microgravity culturing, cells are often initially sown out onto small (ca. 100 µm diameter) beads (this accelerates the formation of microtissue structures but is not essential). As prototissues are formed by cell growth around these beads, the beads are often ejected or become completely covered with cells. Prototissues formed in this manner become very highly differentiated so as to resemble adult tissue.

Microgravity bioreactors have been used in a variety of contexts. Early studies showed that microgravity bioreactor systems helped cells form three dimensional structures by reducing shear stress on the cells [Reduced shear stress: a major component in the ability of mammalian tissues to form three-dimensional assemblies in simulated microgravity. Goodwin TJ, Prewett TL, Wolf DA, Spaulding GF. J Cell Biochem. 1993 Mar;51 (3):301-11.

Now a significant body of literature demonstrates increased differentiation of cells grown in a microgravity bioreactor system. For a review see: [Growing tissues in microgravity. Unsworth BR, Lelkes PI. Nat Med. 1998 Aug;4(8):901-7.] For example, microgravity culturing induces neural precursor cells to form cellular clusters or "neurospheres". These prototissues are characterized by a crude, but organized, architecture, having a surface layer of immature proliferating cells (nesting- and proliferating cell nuclear antigen-positive) that enclose strata of more differentiated cells (beta-tubulin III- and glial fibrillary acidic protein-positive). These "neurospheres" show promise for development of neurotransplantable tissue. See e.g. [Neural precursor cells form rudimentary tissue-like structures in a rotating-wall vessel bioreactor. Low HP, Savarese TM, Schwartz WJ.In vitro Cell Dev Biol Anim. 2001 Mar;37(3):141-7.] and see [Rapid differentiation of NT2 cells in Sertoli-NT2 cell tissue constructs grown in the rotating wall bioreactor. Saporta S, Willing AE, Shamekh R, Bickford P, Paredes D, Cameron DF. Brain Res Bull. 2004 Dec 150;64(4):347-56.].

Or for another example, microgravity culturing of a multipotential human retinal cell line induced expression of a nearly *in vivo* phenotype, which could not be achieved when the cells were grown under other conditions [Generation of 3D retina-like structures from a human retinal cell line in a NASA bioreactor. Dutt K, Harris-Hooker S, Ellerson D, Layne D, Kumar R, Hunt R. Cell Transplant. 2003;12(7):717-31.]

Some technical problems with microgravity bioreactors have been reported. For example, when temporomandibular joint (TMJ) disc tissues were engineered using either flat culture or a microgravity bioreactor, there were no significant differences in total matrix content and compressive stiffness, notwithstanding marked differences in gross appearance, histological structure, and distribution of collagen types I and II (with the bioreactor discs having more collagen type II). The authors concluded that improvements of the microgravity bioreactor culture system were needed [Detamore MS, Athanasiou KA. Use of a rotating bioreactor toward tissue engineering the temporomandibular joint disc. Tissue Eng. 2005 Jul-Aug;11 (7-8):1188-97].

Although well known and widely used, currently available microgravity bioreactors have significant limitations:

Currently, the minimum size of a microgravity bioreactor is about 10 ml. In many circumstances, a much smaller volume is desirable, in order to reduce consumption of precious materials and/or facilitate "high throughput" testing. For example, a smaller volume bioreactor would reduce amounts of both cells and compounds used in testing pharmaceutical compounds at different concentrations and times. Reduction of materials consumption is especially important in radioactive labelling experiments, where costs and hazards can be especially high. To give a practical example, during investigation of effects of new pharmaceutical compositions on gene expression or other biological responses of cultured cells, multiple concentrations would typically be tested over multiple time points. For statistical power such analyses are typically repeated multiple times. A single experiment would require as much as 2.5 litres of cells, where one composition is tested in 5 replications at 5 concentrations, for 10 time points, using a 10 ml bioreactor. If metabolic incorporation of a radioactive label was required at, for example, 1 mCi/ml, such an experiment would require 2.5Ci of radioactivity. Accordingly, it would be advantageous to provide a microgravity bioreactor with much smaller incubation compartment volume.

Another significant limitation of microgravity bioreactors of the prior art is moisture loss, which affects cell growth. De-hydration (even only by 5-10%) during incubation can result in changes in pH and other concentration-dependent parameters, such as concentrations of salts, nutrient substances, and the like. Many cell types are highly sensitive to their environment. For such cells, even a small change in such environmental conditions can influence cell growth and gene expression. This problem is especially pronounced in a small volume bioreactor, where small changes in volume can cause relatively large changes in concentration-dependent parameters. Without some solution to this de-hydration problem, a small volume bioreactor would experience rapid loss of moisture, notwithstanding maintenance of humidified conditions (100% relative humidity) in the incubator where the bioreactor was used. This tendency for rapid de-hydration in a small volume bioreactor, that is, this tendency for rapid change in relative volume greatly increases the need for time-consuming manual monitoring and manipulation, for example to replenish or exchange culture medium. This tendency effectively renders long-term maintenance of cultures in a small volume bioreactor impractical or impossible. Accordingly, it would be advantageous to provide a microgravity bioreactor with very high relative water retention in the cell incubation compartment.

Still another limitation of microgravity bioreactors of the prior art is that access ports used for adding or removing cells and growth medium have typically relied on conventional "luer lock" closures. These and similar closures have a finite 'dead' volume and this becomes proportionally larger as the volume of the bioreactor is reduced. This disadvantage can be circumvented by using ports of essentially no dead volume.

Luer lock closures can also lead to presence of air bubbles in the incubation compartment. Bioreactors are preferably kept free of air bubbles in the incubation compartment which otherwise have detrimental effects, breaking up the prototissues. This air bubble problem is especially pronounced in a small volume bioreactor, where a single bubble can represent a relatively significant volume. Some solutions to the air bubble problem are known in the prior art. For example, WO 95/07344 provides a reservoir chamber for entrapping gas bubbles away from the incubation compartment. However, these solutions would be wholly unsuitable for a small volume bioreactor because of the volumes involved. A better solution is thus to provide a closure mechanism for access ports that excludes any possibility of introducing air bubbles into the incubation compartment.

Conventional "luer lock" and similar closures also increase fluid turbulence and this can lead to increased shear forces which will have a detrimental effect on the prototissues. Microgravity bioreactors require continuous rotation of the incubation compartment to maintain microgravity conditions for differentiated or differentiating cells and other tissues. If the incubation compartment inner surface is not suitably adapted, it may give rise to turbulence. Such turbulence may lead to tearing or "shearing" of prototissues. Accordingly, it is advantageous to provide microgravity bioreactors with an access port closure mechanism that avoids turbulence.

WO 95/07344, US 5153131, US 5437998, US 5665594, US 5989913, and US 6,642,019 each disclose improvements of microgravity bioreactors. US 2005/0084965 discloses use of a conventional, commercially available microgravity bioreactor for incubating hepatocyte spheroids. However none of these patents or published applications addresses the problem of de-hydration or discloses a microgravity bioreactor having a small incubation compartment volume or having a zero volume access port closure.

WO-A-0238735 discloses a bioreactor adapted for rotation comprising a chamber wherein cells are incubated, a chamber for the fluids comprising the nutrients, a semipermeable membrane separating both chambers, means for providing a flow of fluids in the second chamber. The volume of the incubation cavity is not specifically disclosed in WO-A-0238735, but considering the volume of fluids used in the examples it can be assumed that the volume of the incubation chamber is more than 1 ml.

US-A-5702941 describes a bioreactor adapted for rotation, comprising an incubation chamber, a fluid chamber, means for establishing a fluid flow, and a semipermeable membrane. The volume of the whole vessel (both chambers) is at least 1 ml.

Hence, the prior art does not describe a bioreactor adapted for rotation comprising an incubation chamber with a volume less than 0,5 ml.

Accordingly, it is advantageous to provide an improved microgravity bioreactor that addresses these problems.

### Summary of the invention

In preferred embodiments, the invention provides a bioreactor that mitigates, alleviates eliminates or otherwise solves one or more of the above mentioned problems of the prior art.

This and several other objectives are obtained in a first aspect of the invention by providing a bioreactor adapted for rotation, the reactor comprising
- an incubation cavity, the incubation cavity providing, in conjunction with a first semipermeable filter, a substantially closed confinement, said semipermeable filter being permeable to molecules up to a predetermined molecular weight or size, allowing an inflow of nutrients and fluid culture medium in the incubation chamber and retainment of cells and cellular aggregates in the incubation cavity,
- an equilibrium chamber, said equilibrium chamber providing a volume for a supply of nutrient factors and/or oxygen and optionally carbon dioxide and/or pH controlling substances for one or more cell cultures, tissue biopsies, or cell clusters resident in the incubation cavity, and
- conduction means providing a substantially fluid tight conduit from the semipermeable filter to the equilibrium chamber,
wherein the incubation cavity has an internal fluid volume of less than 500 µL.

In a second aspect, embodiments of the invention provide a bioreactor adapted for rotation, the bioreactor comprising
- an incubation cavity, the incubation cavity providing, in conjunction with a first semipermeable membrane, a substantially closed confinement,
- a humidity chamber comprising an aqueous liquid, the chamber comprising a second semipermeable membrane and a third semipermeable membrane both arranged in fluid contact with the aqueous liquid, said third permeable membrane further being arranged for exchange of gases with an atmosphere surrounding the bioreactor, and
- conduction means providing a substantially air tight conduit from the first semipermeable membrane to the second semipermeable membrane,
wherein the first, second and third semipermeable membranes are substantially impermeable to water and substantially permeable to oxygen and carbon dioxide so as to facilitate:
a) aeration of the incubation cavity through the first semipermeable membranes and the humidity chamber, and
b) substantial retainment of water in the incubation chamber. optionally wherein the incubation cavity has an internal fluid volume of less than 500 µL.

In a third aspect, embodiments not being part of the claimed invention provide a bioreactor adapted for rotation, the bioreactor comprising
- an incubation cavity, the cavity comprising an inner surface in conjunction with a flexible membrane or a flexible filter,
- one or more through-going ports adapted for filling of the incubation cavity, said ports being arranged azimuthally at different angles as viewed from a central axis of the incubation cavity,
- one or more inserts, each insert being adapted to fit in a corresponding through-going port to provide a substantially fluid tight lock of each port,
wherein one or more of the inserts has an end portion that is substantially aligned with the inner surface of the incubation cavity when inserted in the corresponding port thereby creating an essentially zero volume port,
optionally wherein the incubation cavity has an internal fluid volume of less than 500 µL.

In a fourth aspect, embodiments not being part of the claimed invention provide a bioreactor adapted for rotation, the bioreactor comprising
- an incubation cavity, the incubation cavity providing, in conjunction with a semipermeable membrane, a substantially closed confinement,
wherein at atmospheric pressure the semipermeable membrane is almost completely impermeable to water while substantially permeable to oxygen and carbon dioxide so as to facilitate:
a) aeration of the incubation cavity through the first semipermeable membranes, and
b) substantial retainment of water in the incubation chamber. optionally wherein the incubation cavity has an internal fluid volume of less than 500 µL.

In a fifth aspect, embodiments not being part of the claimed invention provide use of the bioreactor for the incubation of one or more cell cultures, tissue biopsies, cell clusters, tissue-like structures, "prototissues" or similar samples.

In a sixth aspect, embodiments not being part of the claimed invention provide a method of incubating one or more cell types tissue biopsies, cell clusters, tissue-like structures, "prototissues" or similar samples.

In a seventh aspect, embodiments not being part of the claimed invention provide a method of creating molecular profiles of the biological effect of chemical compositions.

In an eighth aspect, embodiments not being part of the claimed invention provide a method of compiling a library of molecular profiles of the biological effect of chemical compositions having predetermined toxicities.

In a ninth aspect, embodiments not being part of the claimed invention provide a method of typing toxicity of a test chemical composition.

Preferred embodiments of the present invention are particularly, but not exclusively, advantageous for obtaining a microgravity bioreactor that provides:
1) Reduced consumption of resources (chemicals, cells or tissues) with corresponding reduction of cost, due to a smaller incubation compartment volume.
2) Improved moisture retention in the incubation chamber with corresponding improved maintenance of culture conditions for long-term growth, due to introduction of a humidity chamber.
3) Increased duration of long-term cultures incubated in a small volume bioreactor, due to improved moisture retention in the incubation chamber.
4) Reduced fluid turbulence, due to introduction of essentially zero-volume access ports.
5) Additional benefits of smaller bioreactor volume including improved monitoring of cell growth, in some embodiments by automatic video/camera imaging techniques; improved attainment of biological threshold levels of growth factors and signal molecules using smaller samples of selected biopsies or cultures; increased efficiency of standard sized incubators, due to the increased number of individual bioreactors that can be operated simultaneously.

The first, second, third, fourth, fifth, sixth, seventh, eighth and ninth aspect of embodiments may each be combined with any of the other aspects. These and other aspects of the invention will be apparent from and elucidated with reference to the embodiments described hereinafter.

### Brief description of the Figures

Some embodiments of the present invention are illustrated by the accompanying Figures, corresponding only figures 1, 2, 3A, 3B, 4, 5, 7, 13 and 15 to embodiments of the invention and where
Figure 1 is a schematic cro'ss-sectional drawing of a bioreactor according to the first Figure 1 is a schematic cross-sectional drawing of a bioreactor according to the first aspect of the invention,
Figure 2 is a more detailed cross-sectional drawing of a bioreactor according to the first aspect of the invention showing *inter alia* pumping means,
Figure 3A is a schematic cross-sectional drawing of a bioreactor according to the second aspect of the invention,
Figure 3B is a schematic cross-sectional drawing of an alternative embodiment of a bioreactor according to the second aspect of the invention (where the membrane used is totally impervious to salts but still pervious to gasses),
Figure 4 is a plane view (A) and a cross-sectional view (B) of a humidity chamber of a bioreactor according to the second aspect of the invention,
Figure 5 is a more detailed cross-sectional drawing of a bioreactor according to the second aspect of the invention showing *inter alia* air inlets,
Figure 6 is a schematic cross-sectional drawing of a bioreactor according to the fourth aspect,
Figure 7 is a graph showing experimental results of the liquid loss under long-term incubation with and without the humidity chamber of the bioreactor according to the second aspect of the invention,
Figure 8 is a cross-sectional drawing of a bioreactor according to the third aspect,
Figure 9 is another cross-sectional drawing of a bioreactor according to the third aspect,
Figure 10 is a schematic drawing of a portion of the inner surface of the bioreactor and inserts for closing the bioreactor,
Figure 11, A to D, is a sequence of schematic drawings illustrating a method for operating a bioreactor according to the third aspect, and
Figure 12 shows a cross-sectional side view (part A) and a cross-sectional front view (part B), respectively, of a mount for a bioreactor according to the third aspect.
Figure 13 is another cross-sectional drawing of a bioreactor according to the second aspect of the invention.
Figure 14 is another cross-sectional drawing of a bioreactor according to the third aspect.
Figure 15 is a plane view (A) and cross-sectional view (B) and (C) of a bioreactor according to the second aspect of the invention.

### Detailed description

### Definitions

As used herein, the following terms have the following meanings:

The terms "semipermeable filter" and "semipermeable membrane" refer to a filter or membrane that can be penetrated by some, but not all, chemical or biological substances. The terms are used interchangeably except that, in general, "filter" is used where water is freely permeable while, in general, "membrane" is used both where water is not freely permeable and also where water is freely permeable.

The term "incubation cavity" refers to that portion of a bioreactor in which cell cultures, tissue biopsies, cell clusters, tissue-like structures, "prototissues" or similar samples are grown, differentiated, incubated, or otherwise cultured. The term "incubation cavity" is used interchangeably with "incubation chamber" and "incubation compartment."

The term "substantially impermeable to water" is used to describe characteristics of membranes of the present invention and refers to a membrane that exhibits a high degree of repulsion of water and water-like molecules in gas and/or liquid phase.

The term "almost completely impermeable to water" is used to describe characteristics of membranes of the present invention and refers to a membrane across which the water flow rate at 1 bar is not greater than 0.1 mL/min/cm².

The term "substantially permeable to oxygen and carbon dioxide" is used to describe characteristics of membranes of the present invention and refers to a membrane across which air will readily pass.

The term "relative retainment" is used to describe conditions arising from operation of a bioreactor of the invention with an aqueous solution or suspension in the incubation cavity and refers to the relative amount of residual substance initially present. For example, the relative retainment of water in the incubation cavity (with a flexible membrane) may be calculated as the volume of the cavity after operating the bioreactor divided by the volume of the cavity at the beginning of operating the bioreactor.

The term "toxic" has the usual meaning known in the art. A "toxic" substance is a substance that in the amount present in the chemical compositions as defined above can impair the functioning of, or cause structural damage to a cell, tissue or organism.

The term "predetermined toxicity" relates to both toxic and non-toxic substances. As Paracelsus stated in the 16^{th} century, "All things are poison and nothing is without poison, only the dose permits something not to be poisonous". The toxicity type of a substance may e.g. be determined according to the toxicity typing scheme of the Food and Drug Administration (FDA) of the United States of America. According to this scheme, the predetermined toxicity of a substance may belong to toxicity type A, B, etc. or may be non-toxic.

The term "cell cultures" refers to any kind of cells, tissue biopsies, cell clusters, tissue-like structures, "prototissues" or similar samples obtained or initially cultured by any method known in the art.

The term "microgravity bioreactor" refers to a bioreactor adapted for rotation.

The term "incubating under microgravity conditions" refers to growth of cell cultures in a bioreactor adapted for rotation while rotating said bioreactor about a substantially horizontal central axis at a rate that suspends one or more cell cultures in a liquid culture medium and continuing such rotation for a time period that permits growth of said one or more cell cultures.

The term "means of relative retainment of water" is used to describe features of a bioreactor and refers to any means other than perfusion that is used in combination with a membrane or filter that substantially confines the incubation chamber to achieve relative retainment of water in the incubation chamber or, in the alternative, to any single membrane that substantially confines the incubation chamber across which membrane the water flow rate at 1 bar is not greater than 0.1 mL/min/cm². The term "chemical composition" has the usual meaning known in the art. It may include, but is not limited to, any mixture of one or more chemical or biological agents, such as small molecules, peptides, proteins, bases, nucleic acids, and lipids, wherein said chemical or biological agents result in alterations in gene expression or protein expression in one or more cell type selected from the group consisting of:
o Keratinizing epithelial cells (including Epidermal keratinocyte (differentiating epidermal cell); Epidermal basal cell (stem cell); Keratinocyte of fingernails and toenails, Nail bed basal cell (stem cell); Medullary hair shaft cell; Cortical hair shaft cell; Cuticular hair shaft cell; Cuticular hair root sheath cell; Hair root sheath cell of Huxley's layer; Hair root sheath cell of Henle's layer; External hair root sheath cell; Hair matrix cell (stem cell)).
o Wet stratified barrier epithelial cells (including Surface epithelial cell of stratified squamous epithelium of cornea, tongue, oral cavity, esophagus, anal canal, distal urethra and vagina; basal cell (stem cell) of epithelia of cornea, tongue, oral cavity, esophagus, anal canal, distal urethra and vagina; Urinary epithelium cell (lining urinary bladder and urinary ducts)).
o Exocrine secretory epithelial cells (including Salivary gland mucous cell (polysaccharide-rich secretion); Salivary gland serous cell (glycoprotein enzyme-rich secretion); Von Ebner's gland cell in tongue (washes taste buds); Mammary gland cell (milk secretion); Lacrimal gland cell (tear secretion); Ceruminous gland cell in ear (wax secretion); Eccrine sweat gland dark cell (glycoprotein secretion); Eccrine sweat gland clear cell (small molecule secretion); Apocrine sweat gland cell (odoriferous secretion, sex-hormone sensitive); Gland of Moll cell in eyelid (specialized sweat gland); Sebaceous gland cell (lipid-rich sebum secretion); Bowman's gland cell in nose (washes olfactory epithelium); Brunner's gland cell in duodenum (enzymes and alkaline mucus); Seminal vesicle cell (secretes seminal fluid components, including fructose for swimming sperm); Prostate gland cell (secretes seminal fluid components); Bulbourethral gland cell (mucus secretion); Bartholin's gland cell (vaginal lubricant secretion); Gland of Littre cell (mucus secretion); Uterus endometrium cell (carbohydrate secretion); Isolated goblet cell of respiratory and digestive tracts (mucus secretion); Stomach lining mucous cell (mucus secretion); Gastric gland zymogenic cell (pepsinogen secretion); Gastric gland oxyntic cell (hydrochloric acid secretion); Pancreatic acinar cell (bicarbonate and digestive enzyme secretion); Paneth cell of small intestine (lysozyme secretion); Type II pneumocyte of lung (surfactant secretion); Clara cell of lung).
o Hormone secreting cells (including Anterior pituitary cells; Somatotropes; Lactotropes; Thyrotropes; Gonadotropes; Corticotropes; Intermediate pituitary cell, secreting melanocyte-stimulating hormone; Magnocellular neurosecretory cells secreting oxytocin or vasopressin; Gut and respiratory tract cells (secreting one or more of the following: serotonin, endorphin, somatostatin, gastrin, secretin, cholecystokinin, insulin, glucagon, bombesin); Thyroid gland cells; thyroid epithelial cell; parafollicular cell; Parathyroid gland cells; Parathyroid chief cell; oxyphil cell; Adrenal gland cells; chromaffin cells (secreting one or more steroid hormones mineralcorticoids or glucocorticoids); Leydig cell of testes (secreting testosterone); Theca interna cell of ovarian follicle (secreting oestrogen); Corpus luteum cell of ruptured ovarian follicle (secreting progesterone); Kidney juxtaglomerular apparatus cell (secreting renin); Macula densa cell of kidney; Peripolar cell of kidney; Mesangial cell of kidney).
o Epithelial absorptive cells (Gut, Exocrine Glands and Urogenital Tract) (including Intestinal brush border cell (with microvilli); Exocrine gland striated duct cell; Gall bladder epithelial cell; Kidney proximal tubule brush border cell; Kidney distal tubule cell; Ductulus efferens nonciliated cell; Epididymal principal cell; Epididymal basal cell; Metabolism and storage cells; Hepatocyte (liver cell); Adipocyte (white or brown fat cell); Liver lipocyte).
o Barrier function cells (Lung, Gut, Exocrine Glands and Urogenital Tract) (including Type I pneumocyte (lining air space of lung); Pancreatic duct cell (centroacinar cell); Nonstriated duct cell (of sweat gland, salivary gland, mammary gland, etc.); Kidney glomerulus parietal cell; Kidney glomerulus podocyte; Loop of Henle thin segment cell (in kidney); Kidney collecting duct cell; Duct cell (of seminal vesicle, prostate gland, etc.)).
Epithelial cells lining closed internal body cavities (including Blood vessel and lymphatic vascular endothelial fenestrated cell; Blood vessel and lymphatic vascular endothelial continuous cell; Blood vessel and lymphatic vascular endothelial splenic cell; Synovial cell (lining joint cavities, hyaluronic acid secretion); Serosal cell (lining peritoneal, pleural, and pericardial cavities); Squamous cell (lining perilymphatic space of ear); Squamous cell (lining endolymphatic space of ear); Columnar cell of endolymphatic sac with microvilli (lining endolymphatic space of ear); Columnar cell of endolymphatic sac without microvilli (lining endolymphatic space of ear); Dark cell (lining endolymphatic space of ear); Vestibular membrane cell (lining endolymphatic space of ear); Stria vascularis basal cell (lining endolymphatic space of ear); Stria vascularis marginal cell (lining endolymphatic space of ear); Cell of Claudius (lining endolymphatic space of ear); Cell of Boettcher (lining endolymphatic space of ear); Choroid plexus cell (cerebrospinal fluid secretion); Pia-arachnoid squamous cell; Pigmented ciliary epithelium cell of eye; Nonpigmented ciliary epithelium cell of eye; Corneal endothelial cell).
o Ciliated cells with propulsive function (including Respiratory tract ciliated cell; Oviduct ciliated cell (in female); Uterine endometrial ciliated cell (in female); Rete testis cilated cell (in male); Ductulus efferens ciliated cell (in male); Ciliated ependymal cell of central nervous system (lining brain cavities)).
o Extracellular matrix secretion cells (including Ameloblast epithelial cell (tooth enamel secretion); Planum semilunatum epithelial cell of vestibular apparatus of ear (proteoglycan secretion); Organ of Corti interdental epithelial cell (secreting tectorial membrane covering hair cells); Loose connective tissue fibroblasts; Corneal fibroblasts; Tendon fibroblasts; Bone marrow reticular tissue fibroblasts; Other nonepithelial fibroblasts; Blood capillary pericyte; Nucleus pulposus cell of intervertebral disc; Cementoblast/cementocyte (tooth root bonelike cementum secretion); Odontoblast/odontocyte (tooth dentin secretion); Hyaline cartilage chondrocyte; Fibrocartilage chondrocyte; Elastic cartilage chondrocyte; Osteoblast/osteocyte; Osteoprogenitor cell (stem cell of osteoblasts); Hyalocyte of vitreous body of eye; Stellate cell of perilymphatic space of ear).
o Contractile cells (including Red skeletal muscle cell (slow); White skeletal muscle cell (fast); Intermediate skeletal muscle cell; nuclear bag cell of Muscle spindle; nuclear chain cell of Muscle spindle; Satellite cell (stem cell); Ordinary heart muscle cell; Nodal heart muscle cell; Purkinje fiber cell; Smooth muscle cell (various types); Myoepithelial cell of iris; Myoepithelial cell of exocrine glands).
o Blood and immune system cells (including Erythrocyte (red blood cell); Megakaryocyte (platelet precursor); Monocyte; Connective tissue macrophage (various types); Epidermal Langerhans cell; Osteoclast (in bone); Dendritic cell (in lymphoid tissues); Microglial cell (in central nervous system); Neutrophil granulocyte; Eosinophil granulocyte; Basophil granulocyte; Mast cell; Helper T cell; Suppressor T cell; Cytotoxic T cell; B cells; Natural killer cell; memory cell; Reticulocyte; Stem cells and committed progenitors for the blood and immune system (various types)).
o Sensory transducer cells (including Photoreceptor rod cell of eye; Photoreceptor blue-sensitive cone cell of eye; Photoreceptor green-sensitive cone cell of eye; Photoreceptor red-sensitive cone cell of eye; Auditory inner hair cell of organ of Corti; Auditory outer hair cell of organ of Corti; Type I hair cell of vestibular apparatus of ear (acceleration and gravity); Type II hair cell of vestibular apparatus of ear (acceleration and gravity); Type I taste bud cell; Olfactory receptor neuron; Basal cell of olfactory epithelium (stem cell for olfactory neurons); Type I carotid body cell (blood pH sensor); Type II carotid body cell (blood pH sensor); Merkel cell of epidermis (touch sensor); Touch-sensitive primary sensory neurons (various types); Cold-sensitive primary sensory neurons; Heat-sensitive primary sensory neurons; Pain-sensitive primary sensory neurons (various types); Proprioceptive primary sensory neurons (various types)).
o Autonomic neuron cells (including Cholinergic neural cell (various types); Adrenergic neural cell (various types); Peptidergic neural cell (various types)).
o Sense organ and peripheral neuron supporting cells (including Inner pillar cell of organ of Corti; Outer pillar cell of organ of Corti; Inner phalangeal cell of organ of Corti; Outer phalangeal cell of organ of Corti; Border cell of organ of Corti; Hensen cell of organ of Corti; Vestibular apparatus supporting cell; Type I taste bud supporting cell; Olfactory epithelium supporting cell; Schwann cell; Satellite cell (encapsulating peripheral nerve cell bodies); Enteric glial cell).
o Central nervous system neurons and glial cells (including Neuron cells (large variety of types, still poorly classified); Astrocyte (various types); Oligodendrocyte).
o Lens cells (including Anterior lens epithelial cell; Crystallin-containing lens fiber cell).
o Pigment cells (including Melanocyte; Retinal pigmented epithelial cell).
o Germ cells (including Oogonium/Oocyte; Spermatid; Spermatocyte; Spermatogonium cell (stem cell for spermatocyte); Spermatozoon).
o Nurse cells (including Ovarian follicle cell; Sertoli cell (in testis); Thymus epithelial cell).
o Stem cells (omnipotent, totipotent, pleuripotent, unipotent stem cells or precursor or progenitor cells, of both embryonic and adult origin).
o All cancer cells (including teratocarcinomas of indefinable origin) derived from any of the above mentioned cell types.

### Preferred embodiments

In preferred embodiments, the semipermeable filters utilised in the present invention allow passage of molecules up to a certain molecular weight or size. Semipermeable filters with a well-defined pore size are known to the person skilled in the art and are commercially available. In preferred embodiments of the invention, semipermeable filters may be permeable to molecules up to a predetermined molecular weight, such as 50 kDa, 100kDa, 150 kDa, 200 kDa or 250 kDa. Alternatively, the permeability of semipermeable filters may be determined by the pore sizes therein. The pore size of semipermeable filters may be less than or equal to 0.5 µm, such as less than or equal to 0.3 µm, preferably less than or equal to 0.2 µm, even more preferably less than or equal to 0:1 µm, and most preferably less than or equal to 0.05 µm. A wide variety of filters can be used. These could be made of materials selected from (but not limited to) the group consisting of polytetrafluroethylene (PTFE), Polyvinylidene fluoride (PVDF), silicon rubber, foam plastics, radiation treated plastic, and similar materials. In one preferred embodiment, a TE 35 filter from Whatman or a Zefluor filter (cat. no. 66142 from Pall Life Sciences can be used.

In preferred embodiments of the invention, the water flow rate at 1 bar accross membranes that are "substantially impermeable to water" and "substantially permeable to oxygen and carbon dioxide" is not greater than 50 ml/min/cm², preferably not greater than 40 ml/min/cm², more preferably not greater than 30 ml/min/cm², even more preferably not greater than 20 ml/min/cm², most preferably not greater than 10 ml/min/cm². It will be readily understood by those skilled in the art that water permeability can be expressed in other units, which can be converted into ml/min/ cm².

In preferred embodiments of the invention, the air flow rate at 3 mbar accross membranes that are "substantially impermeable to water" and "substantially permeable to oxygen and carbon dioxide" is at least 5 ml/min/cm², preferably at least 10 ml/min/cm², more preferably at least 15 ml/min/cm², even more preferably at least 20 ml/min/cm², most preferably at least 25 ml/min/cm². It will be readily understood by those skilled in the art air flow can be expressed in other units, which can be converted into ml/min/ cm².

Membranes comprised of a wide variety of materials can be used, that are "substantially impermeable to water" and "substantially permeable to oxygen and carbon dioxide," including but not limited to membranes well known in the art comprised of polytetrafluoroethylene (PTFE), Polyvinylidene fluoride (PVDF), silicon rubber, foam plastics, radiation treated plastic or similar materials. One example of a suitable membrane is commercially available from Whatman under the trade mark "TE 35®," a PTFE membrane with polyester support having characteristics (quoted by the manufacturer): pore size 0.2µM, thickness 190µM, water flow rate at 0.9bar of 20 ml/min/cm² when measured with ethanol, air flow rate 15 ml/min/cm² at 3 mbar and bubble point 1.4 bar. Another example of a suitable membrane is commercially available from Millipore under the trade mark "SureVent®," a PVDF membrane having characteristics (quoted by the manufacturer): pore size 0.22µM, thickness 100-150µM, water breakthrough 45 mbar, air flow rate >1 slpm /cm² at 10psi. In some embodiments, the membranes can be Millipore 0.22µm "Durapel" membranes or Whatman TE 35 and TE36 membranes.

In preferred embodiments of the invention, the water flow rate at 1 bar accross membranes that are "almost completely impermeable to water" while "substantially permeable to oxygen and carbon dioxide" is not greater than 0.1 ml/min/cm², even more preferably not greater than 0.05 mL/min/cm², still more preferably not greater than 0.04 ml/min/cm², even more preferably not greater than 0.03 ml/min/cm², still more preferably not greater than 0.02 ml/min/cm², most preferably not greater than 0.01 ml/min/cm². It will be readily understood by those skilled in the art that water permeability can be expressed in other units, which can be converted into ml/min/ cm².

In preferred embodiments of the invention, the air flow rate at 3 mbar accross membranes that are "almost completely impermeable to water" while "substantially permeable to oxygen and carbon dioxide" is at least 5 ml/min/cm², preferably at least 10 ml/min/cm², more preferably at least 15 ml/min/cm², even more preferably at least 20 ml/min/cm², most preferably at least 25 ml/min/cm².

Membranes comprised of a wide variety of materials can be used, that are "almost completely impermeable to water" while "substantially permeable to oxygen and carbon dioxide" including but not limited to membranes initially prepared for ultrafiltration purposes that have very low water permeabilities at atmospheric pressures, for example, due to low porosity and high hydrophobicity. Such membranes include ultrafiltration membranes commercially available from Amicon under the trademark "YM1®" and from Pall Corp. under the trademark "Omega 1K. ®". Other suitable membranes include thermoplastic ultrafiltration membranes prepared by thermally induced phase inversion process of semi-crystalline materials such as poly(ether ether ketone)(PEEK) and poly(phenylene sulfide)(PPS), as described by [Micro- and ultrafiltration film membranes from poly(ether ether ketone)(PEEK). Sonnenschein M, Journal of Applied Polymer Science 1999 74:1146]. Immobilized, stable supported liquid membranes (SLM) can also be used comprising a suitable oligomeric or polymeric liquid membrane material immobilized within a solid, microporous, hydrophobic support, such as the system disclosed in US 5507949.

In preferred embodiments, the internal fluid volume of the incubation cavity of a bioreactor according to the present invention is less than 500 uL, may be less than 400 uL, less than 300 uL, less than 200 uL, less than 100 uL, less than 50 uL, or 25 uL.

Many different cell cultures, tissue biopsies, cell clusters, tissue-like structures, "prototissues or similar samples may be used in practising the present invention. Different methods of cell culture include (but are not restricted to) growth in glass and plastic vessels e.g. culture flasks, cell factories or cell cubes and soft plastic bags (like infusion bags) filled with a 3D cell-supporting matrix; roller bottles; spinner bottles; fermentors; or hollow fibres of various materials. The cell cultures may be in the form of cell clusters, such as spheroids on a microcarrier bead, or cells on a scaffold (e.g. biodegradable scaffolds, often made from polyglycolic acid (PGA) PGA, polylactic acid (PLA) or a mixture of the two [Characterization of knitted polymeric scaffolds for potential use in ligament tissue engineering. Ge Z, Goh JC, Wang L, Tan EP, Lee EH. J Biomater Sci Polym Ed. 2005;16(9):1179-92.] and [Synthesis and characterizations of biodegradable and crosslinkable poly(epsilon-caprolactone fumarate), poly(ethylene glycol fumarate), and their amphiphilic copolymer. Wang S, Lu L, Gruetzmacher JA, Currier BL, Yaszemski MJ. Biomaterials. 2005 Aug 12; [Epub ahead of print]], tissue or tissue biopsies, or tissue organoids. The invention may be practised using *inter alia* one or more of the following cell types:
o Keratinizing epithelial cells (including Epidermal keratinocyte (differentiating epidermal cell); Epidermal basal cell (stem cell); Keratinocyte of fingernails and toenails, Nail bed basal cell (stem cell); Medullary hair shaft cell; Cortical hair shaft cell; Cuticular hair shaft cell; Cuticular hair root sheath cell; Hair root sheath cell of Huxley's layer; Hair root sheath cell of Henle's layer; External hair root sheath cell; Hair matrix cell (stem cell)).
o Wet stratified barrier epithelial cells (including Surface epithelial cell of stratified squamous epithelium of cornea, tongue, oral cavity, esophagus, anal canal, distal urethra and vagina; basal cell (stem cell) of epithelia of cornea, tongue, oral cavity, esophagus, anal canal, distal urethra and vagina; Urinary epithelium cell (lining urinary bladder and urinary ducts)).
o Exocrine secretory epithelial cells (including Salivary gland mucous cell (polysaccharide-rich secretion); Salivary gland serous cell (glycoprotein enzyme-rich secretion); Von Ebner's gland cell in tongue (washes taste buds); Mammary gland cell (milk secretion); Lacrimal gland cell (tear secretion); Ceruminous gland cell in ear (wax secretion); Eccrine sweat gland dark cell (glycoprotein secretion); Eccrine sweat gland clear cell (small molecule secretion); Apocrine sweat gland cell (odoriferous secretion, sex-hormone sensitive); Gland of Moll cell in eyelid (specialized sweat gland); Sebaceous gland cell (lipid-rich sebum secretion); Bowman's gland cell in nose (washes olfactory epithelium); Brunner's gland cell in duodenum (enzymes and alkaline mucus); Seminal vesicle cell (secretes seminal fluid components, including fructose for swimming sperm); Prostate gland cell (secretes seminal fluid components); Bulbourethral gland cell (mucus secretion); Bartholin's gland cell (vaginal lubricant secretion); Gland of Littre cell (mucus secretion); Uterus endometrium cell (carbohydrate secretion); Isolated goblet cell of respiratory and digestive tracts (mucus secretion); Stomach lining mucous cell (mucus secretion); Gastric gland zymogenic cell (pepsinogen secretion); Gastric gland oxyntic cell (hydrochloric acid secretion); Pancreatic acinar cell (bicarbonate and digestive enzyme secretion); Paneth cell of small intestine (lysozyme secretion); Type II pneumocyte of lung (surfactant secretion); Clara cell of lung).
o Hormone secreting cells (including Anterior pituitary cells; Somatotropes; Lactotropes; Thyrotropes; Gonadotropes; Corticotropes; Intermediate pituitary cell, secreting melanocyte-stimulating hormone; Magnocellular neurosecretory cells secreting oxytocin or vasopressin; Gut and respiratory tract cells (secreting one or more of the following: serotonin, endorphin, somatostatin, gastrin, secretin, cholecystokinin, insulin, glucagon, bombesin); Thyroid gland cells; thyroid epithelial cell; parafollicular cell; Parathyroid gland cells; Parathyroid chief cell; oxyphil cell; Adrenal gland cells; chromaffin cells (secreting one or more steroid hormones mineralcorticoids or glucocorticoids); Leydig cell of testes (secreting testosterone); Theca interna cell of ovarian follicle (secreting oestrogen); Corpus luteum cell of ruptured ovarian follicle (secreting progesterone); Kidney juxtaglomerular apparatus cell (secreting renin); Macula densa cell of kidney; Peripolar cell of kidney; Mesangial cell of kidney).
o Epithelial absorptive cells (Gut, Exocrine Glands and Urogenital Tract) (including Intestinal brush border cell (with microvilli); Exocrine gland striated duct cell; Gall bladder epithelial cell; Kidney proximal tubule brush border cell; Kidney distal tubule cell; Ductulus efferens nonciliated cell; Epididymal principal cell; Epididymal basal cell; Metabolism and storage cells; Hepatocyte (liver cell); Adipocyte (white or brown fat cell); Liver lipocyte).
o Barrier function cells (Lung, Gut, Exocrine Glands and Urogenital Tract) (including Type I pneumocyte (lining air space of lung); Pancreatic duct cell (centroacinar cell); Nonstriated duct cell (of sweat gland, salivary gland, mammary gland, etc.); Kidney glomerulus parietal cell; Kidney glomerulus podocyte; Loop of Henle thin segment cell (in kidney); Kidney collecting duct cell; Duct cell (of seminal vesicle, prostate gland, etc.)).
o Epithelial cells lining closed internal body cavities (including Blood vessel and lymphatic vascular endothelial fenestrated cell; Blood vessel and lymphatic vascular endothelial continuous cell; Blood vessel and lymphatic vascular endothelial splenic cell; Synovial cell (lining joint cavities, hyaluronic acid secretion); Serosal cell (lining peritoneal, pleural, and pericardial cavities); Squamous cell (lining perilymphatic space of ear); Squamous cell (lining endolymphatic space of ear); Columnar cell of endolymphatic sac with microvilli (lining endolymphatic space of ear); Columnar cell of endolymphatic sac without microvilli (lining endolymphatic space of ear); Dark cell (lining endolymphatic space of ear); Vestibular membrane cell (lining endolymphatic space of ear); Stria vascularis basal cell (lining endolymphatic space of ear); Stria vascularis marginal cell (lining endolymphatic space of ear); Cell of Claudius (lining endolymphatic space of ear); Cell of Boettcher (lining endolymphatic space of ear); Choroid plexus cell (cerebrospinal fluid secretion); Pia-arachnoid squamous cell; Pigmented ciliary epithelium cell of eye; Nonpigmented ciliary epithelium cell of eye; Corneal endothelial cell).
o Ciliated cells with propulsive function (including Respiratory tract ciliated cell; Oviduct ciliated cell (in female); Uterine endometrial ciliated cell (in female); Rete testis cilated cell (in male); Ductulus efferens ciliated cell (in male); Ciliated ependymal cell of central nervous system (lining brain cavities)).
o Extracellular matrix secretion cells (including Ameloblast epithelial cell (tooth enamel secretion); Planum semilunatum epithelial cell of vestibular apparatus of ear (proteoglycan secretion); Organ of Corti interdental epithelial cell (secreting tectorial membrane covering hair cells); Loose connective tissue fibroblasts; Corneal fibroblasts; Tendon fibroblasts; Bone marrow reticular tissue fibroblasts; Other nonepithelial fibroblasts; Blood capillary pericyte; Nucleus pulposus cell of intervertebral disc; Cementoblast/cementocyte (tooth root bonelike cementum secretion); Odontoblast/odontocyte (tooth dentin secretion); Hyaline cartilage chondrocyte; Fibrocartilage chondrocyte; Elastic cartilage chondrocyte; Osteobfast/osteocyte; Osteoprogenitor cell (stem cell of osteoblasts); Hyalocyte of vitreous body of eye; Stellate cell of perilymphatic space of ear).
o Contractile cells (including Red skeletal muscle cell (slow); White skeletal muscle cell (fast); Intermediate skeletal muscle cell; nuclear bag cell of Muscle spindle; nuclear chain cell of Muscle spindle; Satellite cell (stem cell); Ordinary heart muscle cell; Nodal heart muscle cell; Purkinje fiber cell; Smooth muscle cell (various types); Myoepithelial cell of iris; Myoepithelial cell of exocrine glands).
o Blood and immune system cells (including Erythrocyte (red blood cell); Megakaryocyte (platelet precursor); Monocyte; Connective tissue macrophage (various types); Epidermal Langerhans cell; Osteoclast (in bone); Dendritic cell (in lymphoid tissues); Microglial cell (in central nervous system); Neutrophil granulocyte; Eosinophil granulocyte; Basophil granulocyte; Mast cell; Helper T cell; Suppressor T cell; Cytotoxic T cell; B cells; Natural killer cell; memory cell; Reticulocyte; Stem cells and committed progenitors for the blood and immune system (various types)).
o Sensory transducer cells (including Photoreceptor rod cell of eye; Photoreceptor blue-sensitive cone cell of eye; Photoreceptor green-sensitive cone cell of eye; Photoreceptor red-sensitive cone cell of eye; Auditory inner hair cell of organ of Corti; Auditory outer hair cell of organ of Corti; Type I hair cell of vestibular apparatus of ear (acceleration and gravity); Type II hair cell of vestibular apparatus of ear (acceleration and gravity); Type I taste bud cell; Olfactory receptor neuron; Basal cell of olfactory epithelium (stem cell for olfactory neurons); Type I carotid body cell (blood pH sensor); Type II carotid body cell (blood pH sensor); Merkel cell of epidermis (touch sensor); Touch-sensitive primary sensory neurons (various types); Cold-sensitive primary sensory neurons; Heat-sensitive primary sensory neurons; Pain-sensitive primary sensory neurons (various types); Proprioceptive primary sensory neurons (various types)).
o Autonomic neuron cells (including Cholinergic neural cell (various types); Adrenergic neural cell (various types); Peptidergic neural cell (various types)).
o Sense organ and peripheral neuron supporting cells (including Inner pillar cell of organ of Corti; Outer pillar cell of organ of Corti; Inner phalangeal cell of organ of Corti; Outer phalangeal cell of organ of Corti; Border cell of organ of Corti; Hensen cell of organ of Corti; Vestibular apparatus supporting cell; Type I taste bud supporting cell; Olfactory epithelium supporting cell; Schwann cell; Satellite cell (encapsulating peripheral nerve cell bodies); Enteric glial cell).
o Central nervous system neurons and glial cells (including Neuron cells (large variety of types, still poorly classified); Astrocyte (various types); Oligodendrocyte).
o Lens cells (including Anterior lens epithelial cell; Crystallin-containing lens fiber cell).
o Pigment cells (including Melanocyte; Retinal pigmented epithelial cell).
o Germ cells (including Oogonium/Oocyte; Spermatid; Spermatocyte; Spermatogonium cell (stem cell for spermatocyte); Spermatozoon).
o Nurse cells (including Ovarian follicle cell; Sertoli cell (in testis); Thymus epithelial cell).
o Stem cells (omnipotent, totipotent, pleuripotent, unipotent stem cells or precursor or progenitor cells, of both embryonic and adult origin).
o All cancer cells (including teratocarcinomas of indefinable origin) derived from any of the above mentioned cell types.

In a preferred embodiment of the invention, cells that can be applied in the context of the present invention are selected from the group consisting of hepatocytes, adipocytes, kidney cells, muscle cells, or similar cells, liver tissue, fat tissue (brown or white), liver biopsies, kidney biopsies, muscle biopsies, ovarian follicles, islets of Langerhans, and all cancer cells derived therefrom.

In a particularly preferred embodiment of the invention, cells that can be applied in the context of the present invention are hepatocytes, in particular human hepatocytes.

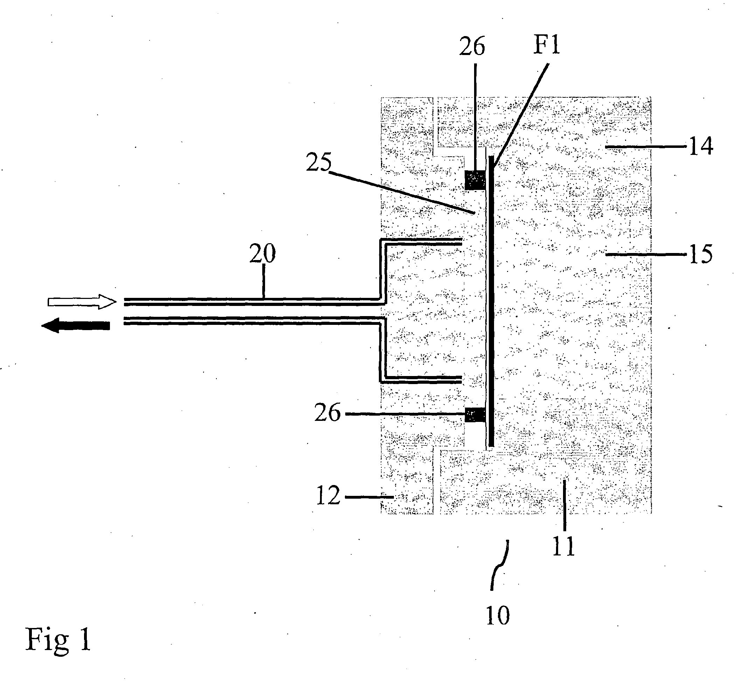

Figure 1 is a schematic cross-sectional drawing of a bioreactor 10 according to the first aspect of the invention. The bioreactor 10 has a high degree of rotational symmetry around a horizontal axis as viewed in Figure 1. The reactor comprises an incubation cavity 15 for incubation of cells, tissues etc. The incubation cavity 15 provides, in conjunction with a first semipermeable filter (also known as a sterile filter) F1, a substantially closed confinement for incubation of cells etc. In order to provide nutrients and/or fresh fluid culture medium, the semipermeable filter F1 is permeable to molecules up to a predetermined molecular weight, such as 50 kDa, 100kDa, 150 kDa, 200 kDa or 250 kDa. Standard dialysis membranes can fulfil these requirements. Alternatively, the permeability of the semipermeable filter F1 is determined by the pore sizes therein. The pore size of the semipermeable filter F1 may be less than or equal to 0.5 µm, such as less than or equal to 0.3 µm, preferably less than or equal to 0.2 µm, even more preferably less than or equal to 0.1 µm, and most preferably less than or equal to 0.05 µm. Sizes less than or equal to 0.2µm are preferable because of the need to prevent infections (e.g. bacteria, mycoplasma or yeasts) entering through the filter. In this preferred embodiment the main purpose of the semipermeable membrane is to allow exchange of nutrients and waste products while excluding cells and bacteria from entering the incubation cavity. Thus a wide variety of filters could be used for F1. These could be made of polytetrafluroethylene (PTFE), Polyvinylidene fluoride (PVDF), silicon rubber, foam plastics, radiation treated plastic or similar materials. Thereby an inflow of nutrients and fluid culture medium into the incubation chamber is provided while at the same time providing retainment of cells and cellular aggregates and their protection from external infection in the incubation cavity 15. The incubation cavity 15 has an internal fluid volume of about 25 µl to about 1 ml. Preferably, the fluid volume of the incubation cavity 15 is about 50 µl to about 0.5 ml, more preferably about 0.1 to about 0.4 ml. The small size significantly reduces the cost of use and the amount of materials (both organic and inorganic) necessary for successful operation. The small size will facilitate close-up monitoring of the cells (e.g. by remote camera), and automated processing (e.g. medium exchange in the non-perfused versions)

An equilibrium chamber (not shown in Figure 1) provides a volume for an exchange of nutrient factors and/or oxygen and optionally carbon dioxide and/or pH controlling substances for one or more cell cultures, tissue biopsies, or cell clusters resident in the incubation cavity 15. Conduction means 20, e.g. tubes and tubular cavities within the back part 12 of the bioreactor 10, provide a substantially fluid tight conduit from the semipermeable filter F1 to the equilibrium chamber. The white arrow indicates fresh culture medium with nutrients and the black arrow indicates used fluid culture medium to be returned to the equilibrium chamber (or to waste disposal).

In the embodiment shown in Figure 1, the conduction means 20 also comprises a media chamber 25 in front of the filter F1 in order to enhance the area of fluid contact between the conduction means 20 and the filter F1 and thereby increase the flow of nutrients and/or fresh fluid culture medium across the filter F1. The media chamber 25 may be provided by a specially designed annulus 26 surrounding the media chamber 25. The annulus 26 could be a sealing O-ring clamped between the back part 12 and a front part 11 of the bioreactor 10. Similarly, the filter F1 is preferably clamped between the back part 12 and the front part 11 to provide a fluid tight and airtight conjunction from the media chamber 25 to the incubation cavity 15. For reasons of clarity small air gaps are visible between the annulus 26 and the filter F1, and between the outer portions of the back part 12 and the outer portions of the front part 11, but when assembled these air gaps are normally absent, because the bioreactor 10 should be as air tight as possible to eliminate fluid leakage.

In the front of the bioreactor 10, a transparent section 14 is located so that the cultivation of cells etc. may be monitored and assessed visually, either manually or automatically with e.g. a camera, from outside of the bioreactor 10. The transparent section 14 could be made of glass, plastic or any other suitable materials being both transparent and biologically and chemically inert with respect to the cultivation process. Preferred materials would include (but not be limited to) various types of glass, polystyrene, polycarbonate, polypropylene, polyethylene and Polymethyl methacrylate (PMMA). Suitable variants of polymethyl methacrylate (PMMA) are available commercially including products marketed under the trademarks/trade names Perspex®, Plexiglas®, Lucite®, Acrylite®, Rhoplex®, and Oroglas®. Any embodiment of the bioreactor could be made in whole or in part from such transparent materials.

The incubation cavity 15 preferably has a substantially cylindrical shape but other shapes are also possible, e.g. elliptical shapes, spherical shapes etc. Preferably, the bioreactor 10 is adapted for rotation around a horizontal, rotational axis by associated rotation means (not shown) to facilitate growth of the cells in the cavity 15. The rate of rotation is adjusted to maintain the cells or prototissues in suspension and this rate has to be varied as the size of the prototissues increases. The person skilled in the art will know how to adjust the rotation speed in order to maintain the cells or prototissues in suspension.

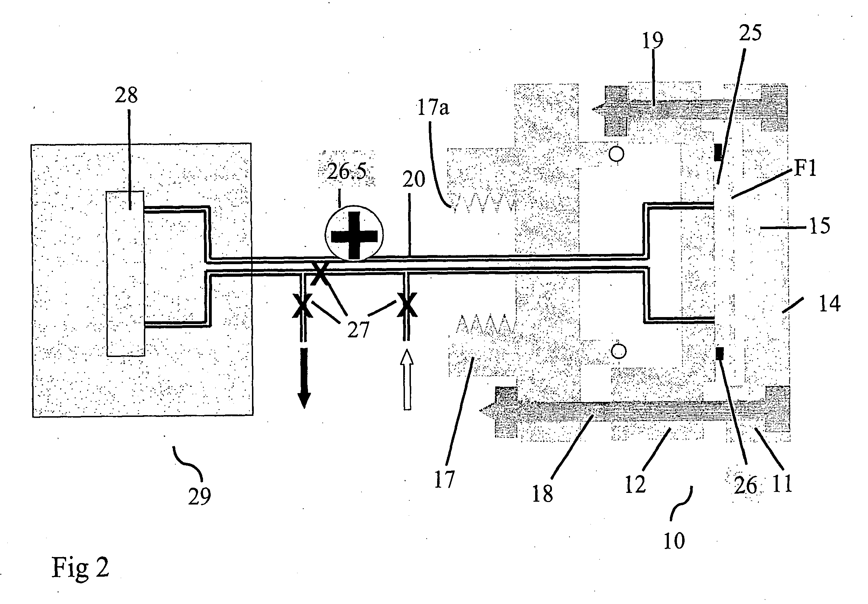

Figure 2 is a more detailed cross-sectional drawing of a bioreactor 10 according to the first aspect of the invention showing also pumping means 26.5 for flowing the fluid culture medium through the conduction means 20, a equilibrium chamber 28 embedded in a constant environment chamber 29, control valves 27 for controlling the flow of fluid culture medium to and from the media chamber 25, to allow the introduction of fresh media (not necessarily the same as that already present, i.e. for the introduction of a compound for testing) and the voiding to waste of spent medium and out of the conduction means 20. The constant environment chamber 29 comprising the equilibrium chamber 28 may be adapted for rotation synchronously with the bioreactor 10 or the container 29 may be fixed in a position next to the bioreactor 10. In the latter case, twisting of the conduction means 20 has to be avoided by incorporating fully rotationally joint readily available and well known to the skilled person into the conduction means 20. It may be preferred that the fluid conduction means 20 has a portion substantially parallel to the rotational axis of the bioreactor 10 to ease design of the overall set-up.

The bioreactor 10 comprising the front part 11 and the back part 12 is to be mounted on a base part 17 by appropriate fastening means, e.g. a through going assembly screw 18. For easy mounting of the base part 17 and the bioreactor 10 on suitable rotation means (not shown), the base part 17 may advantageously have a threaded end section 17a. Similarly, the front part 11 and the back part 12 are additionally held together by an assembly screw 19 in order to provide a fluid tight conjunction to the cavity 15 as explained above. For reasons of clarity small air gaps are visible between the annulus 26 and the filter F1, between the outer portions of the back part 12 and the outer portions of the front part 11, and between the base part 17 and the back part 12, but when assembled these air gaps are normally absent.

In an embodiment of the invention, the conduction means 20 has an internal fluid volume of about 25 µl to about 2 ml, preferably about 50 µl to about 1 ml, most preferably about 0.1 - 0.4 ml. The volume of conductions means 20 should be minimal and is thereby comparable to the volume of the cavity 15. Otherwise, the reduction provided by the present invention in the usage of fluid culture medium and/or relatively expensive reagents (e.g. radioisotopes) may not be significant. Similarly, the equilibrium chamber 28 may advantageously have an internal fluid volume of about 25 µl to about 2 ml, preferably about 50 µl to about 1 ml, most preferably about 0.1 - 0.4 ml.

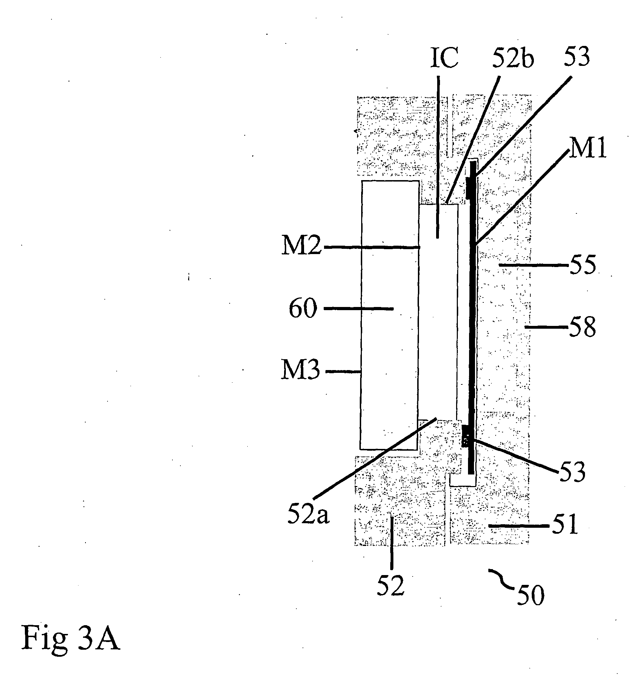

Figure 3A is a schematic cross-sectional drawing of a bioreactor 50 according to the second aspect of the invention. The bioreactor 50 has a high degree of rotational symmetry around a horizontal axis as viewed in Figure 1. The bioreactor comprises an incubation cavity 55 positioned in conjunction with a first semipermeable membrane M1, thereby providing a substantially closed confinement, wherein incubation of cells, tissue and similar may be take place. Incubation of the cells may be may be monitored and assessed visually, either manually or automatical, from outside of the bioreactor 50. The transparent section 58 could be made of glass, plastic or any other suitable materials being both transparent and biological and chemical inert relative to the cultivation process. Preferred materials would include (but not be limited to) various types of glass, polystyrene, polycarbonate, polypropylene, polyethylene and Polymethyl methacrylate (PMMA). Suitable variants of polymethyl methacrylate (PMMA) are available commercially including products marketed under the trademarks/trade names Perspex®, Plexiglas®, Lucite® Acrylite®, Rhoplex®, and Oroglas®.

Additionally, there is a provided a humidity chamber 60 that comprises an aqueous liquid, the chamber 60 further comprises a second semipermeable membrane M2 and a third semipermeable membrane M3. The latter membranes M2 and M3 are both arranged in fluid contact with the aqueous liquid and confine the liquid from the right and left side, respectively, as shown in Figure 1. The third permeable membrane M3 is further arranged for exchange of gases with an atmosphere surrounding the bioreactor 50. The rear part 52 of the bioreactor 50 has a recession fitted to receive the humidity chamber 60 so as to provide a substantially air tight closing of the intermediate cavity IC from the left as shown in Figure 1. The rear part 52 is further arranged with sidewalls 52a and 52b that provide conduction means for a substantially airtight conduit from the first semipermeable membrane M1 to the second semipermeable membrane M2. Thus, the front part 51 of the reactor 50 and the rear part 52 may be manufactured in inert plastic such as high-density poly propylene (HDPP) or similar. Preferred materials would include (but not be limited to) various types of glass, nylon, plastic, polyvinylchloride, polystyrene, polycarbonate, polypropylene, polyethylene and Polymethyl methacrylate. In combination with sealing means 53, e.g. O-shaped sealing ring, the air flow through the intermediate cavity IC is effectively only possible through the first membrane M1 (forming part of the incubation cavity 55) and the second and third membranes M2 and M3 (forming part of the humidity chamber 60).

The first M1, second M2, and third M3 semipermeable membranes are particular in that these membranes are substantially impermeable to water and substantially permeable to oxygen and carbon dioxide. Thereby the membranes M1, M2 and M1 facilitate aeration of the incubation cavity 55 through the first semipermeable membranes M1 and the humidity chamber 60 (traversing M2, the aqueous liquid, and M3). Furthermore, there is achieved a substantial retainment of water in the incubation chamber due to the impermeability for water of the three membranes M1, M2 and M3. The membranes M1, M2 and M3 can be manufactured from a variety of materials well known in the art including but not limited to polytetrafluoroethylene (PTFE), Polyvinylidene fluoride (PVDF), silicon rubber, foam plastics, radiation treated plastic or similar materials. One example of a suitable membrane is commercially available from Whatman under the trade mark "TE 35®," a PTFE membrane with polyester support having characteristics (quoted by the manufacturer): pore size 0.2µM, thickness 190µM, water flow rate at 0.9bar of 20 ml/min/cm² when measured with ethanol, air flow rate 15 ml/min/cm² at 3 mbar and bubble point 1.4 bar. Another example of a suitable membrane is commercially available from Millipore under the trade mark "SureVent®," a PVDF membrane having characteristics (quoted by the manufacturer): pore size 0.22µM, thickness 100-150µM, water breakthrough 45 mbar, air flow rate >1 slpm /cm² at 10psi. Other examples are Zefluor filters (cat. no. 66142 from Pall Life Sciences

Due to the highly hydrophobic nature of these materials water and water-like molecules will to a high degree be repelled from the surface of the membranes M1, M2 and M3. However, some water will inevitably penetrate through the membranes M1, M2 and M3 and this evaporated water from the humidity chamber 60 and/or the incubation chamber 55 will provide a relative humidity of at least 50%, preferably at least 70%, or even more preferably at least 90%, such as at least 95%, 96%, 97%, 98% or 99%, in the air tight conduit formed by the intermediate cavity IC.

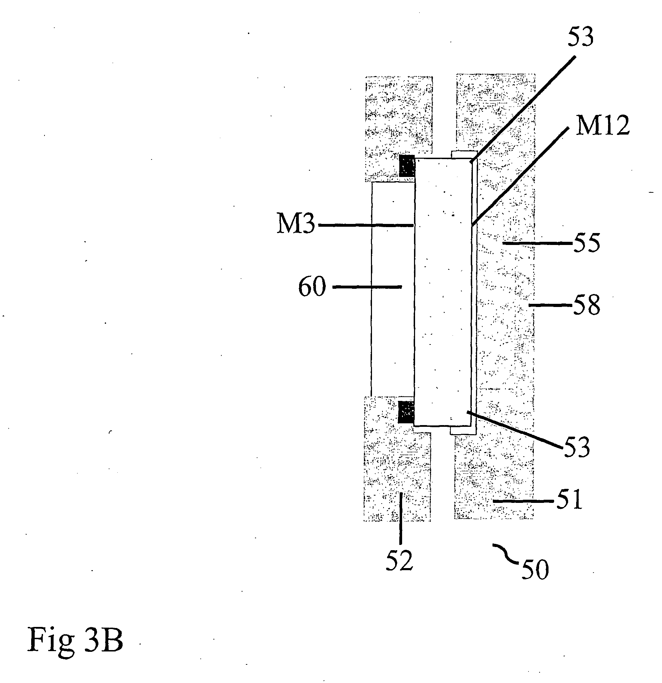

Figure 3B shows an alternative embodiment of a bioreactor according to the second aspect of the invention, wherein the membranes M1 and M2 and the intermediate cavity IC may be replaced by a membrane M12 that is impervious to salts and pervious to gasses and liquids. Examples of this type of film membrane are commercially available including for example products available from Dupont such as polytetrafluoroethylene marketed under the trade mark "Teflon®," FEP membrane or polyvinyl fluoride film marketed under the trade mark "Tedlar®."

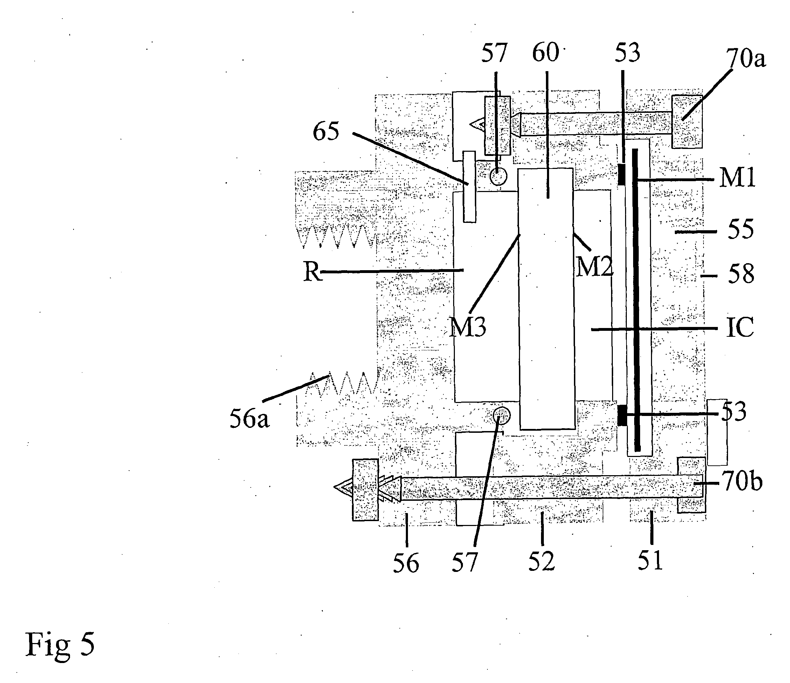

This embodiment is simpler in construction than the embodiment shown in Figure 5 and the humidity chamber is in direct contact with the incubation chamber. One or more cell cultures that may be cultivated in the incubation chamber are very sensitive to changes in the conditions in the incubation chamber, including the amount of salts present. It is therefore important that the membrane M12 is impervious to salts so that the salt level of the incubation chamber remains substantially constant while retaining the amount of liquid in the incubation chamber.

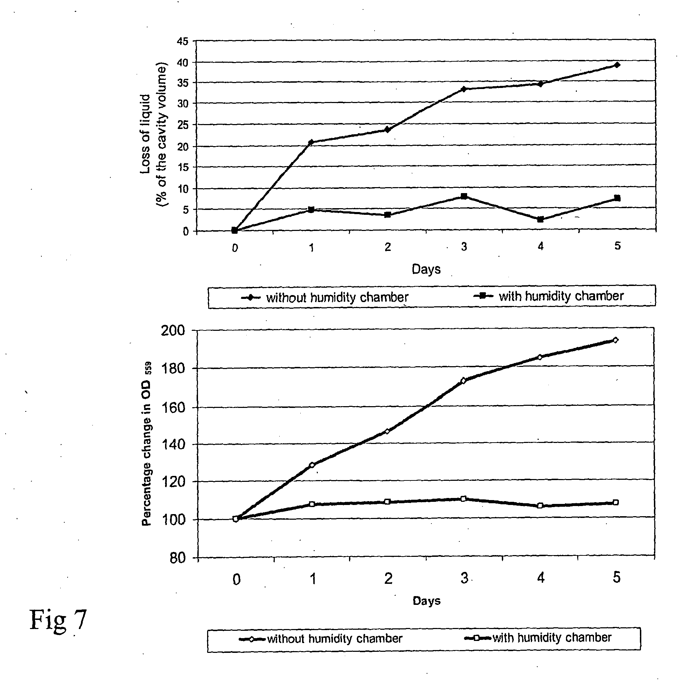

The bioreactor 50 is typically operated at 37°C (for human cells) with an aqueous solution or suspension in the incubation cavity 55, the bioreactor 50 being heated by one or more thermostat controlled heating elements, typically in an incubator for cell culture (not shown). As shown in figure 7, initial experiments demonstrate that the relative retainment of water in the incubation cavity 55 may be at least 80%, preferably at least 90%, or even more preferably at least 95%, such as at least 96%, 97%, 98% or 99%, after 3 days, more preferably after 5 days, or even more preferably after 10 days. Preliminary results also indicate that cell cultures, tissue biopsies, cell clusters, tissue-like structures, "prototissues or similar samples may be incubated for an extended period of time, preferably at least 1 month, such as 2 months, most preferably at least 10 months. Accordingly, by practice of the present invention, cell cultures can be maintained in a bioreactor for prolonged periods of time with minimal change of concentration-dependent aspects of the aqueous growth medium arising from evaporation.

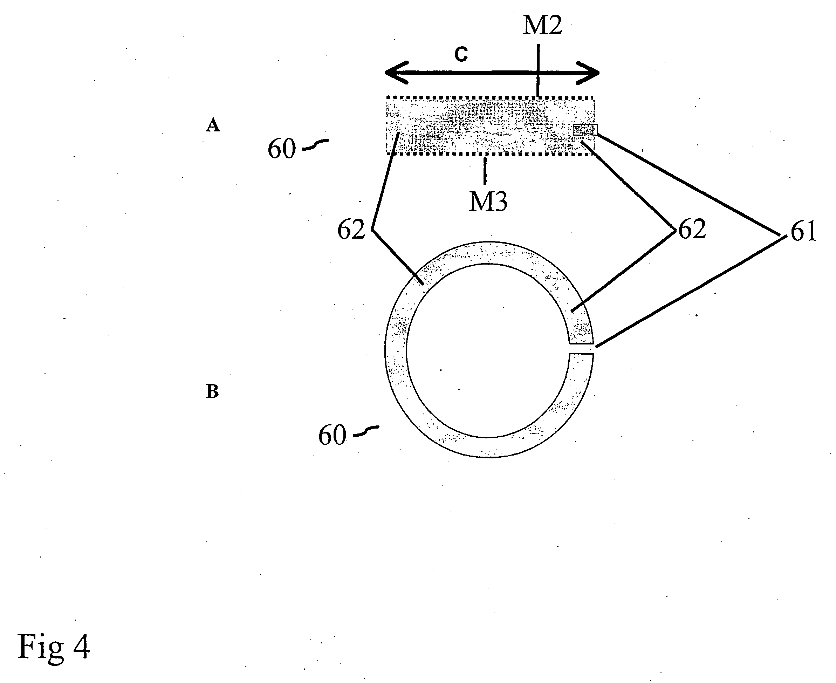

Figure 4 is a plan view (A) and a cross-sectional view (B) of a humidity chamber 60 of a bioreactor 50 according to the second aspect of the invention. In the cross-sectional view in part A, the semipermeable membranes M2 and M3 are shown by dotted lines closing the chamber 60 from above and below as viewed in the cross-sectional view A. The diameter c of the humidity chamber is preferably similar to the diameter of the incubation cavity and thus in the range from 4 to 20 mm, such as such as 6, 8, 10, 12, 14, 16, or 18 mm. The side portions of the chamber 60 are constituted by an annulus 62 manufactured in an inert, stable material such as nylon, plastic, polyvinylchloride, polystyrene, polycarbonate, polypropylene, polyethylene and Polymethyl methacrylate. The annulus 62 may be manufactured in a disposable material so that renewal of the liquid in the humidity chamber 60 during long-term incubation may simply be done by discharging the old humidity chamber 60 and inserting a new humidity chamber 60 in the bioreactor 50. Alternatively or additionally, the humidity chamber 60 may be re-filled with liquid through a filling port 61 closed by e.g. a grub-screw or similar.

Figure 5 is a more detailed cross-sectional drawing of a bioreactor 50 according to the second aspect of the invention showing *inter alia* air inlets 65 and a base part 56 whereupon the bioreactor is mountable. The air inlet 65 supplies a rear cavity R with fresh air. Optionally, the air inlet 65 is connected to or embedded in a gas supply unit (not shown) for providing a controlled atmosphere in the rear cavity R. Thereby the aeration of the cells in the incubation cavity 55 will also be controlled via the air flow passing through the humidity chamber 60. The rear cavity R is constituted by a recession in the base part 56 and sealing means, e.g. a suitable O-ring clamped onto the membrane M3. In order to hold the various parts of the bioreactor 50 firmly together, appropriate fastening means are provided. As shown in Figure 5, a through-going assembly screw 70a keeps the front part 51 and the rear part 52 (and the intermediate positioned sealing 53 and filter M1) together. Similarly, the front part 51 and the rear part 52 are held onto the base part 56 by a through-going screw 70b. Again for reasons of clarity small air gaps are visible between the various parts, e.g. 57 and M3, 52 and 56, 51 and 52, 53 and M1, M1 and 51 and so forth, but when assembled these air gaps are normally absent in order to provide a fluid and air tight closure of the bioreactor 50.

The incubation cavity 55 of Figure 5 has a substantially cylindrical shape having a diameter of the cavity 55 in the range from 4 to 20 mm, such as 6, 8, 10, 12, 14, 16, or 18 mm. The depth of the cavity 55 may be in the range from 2 to 6 mm, such as 2.5, 3, 3.5, 4, 4.5 or 5 mm. Thus, the volume of the cavity 55 is in the range from about 0.03 to 2 ml. Some preferred values of the depth and the diameter, respectively, are 4 mm and 18 mm (resulting in a fluid volume of about 1 ml), 3 mm and 10 mm (resulting in a fluid volume of about 0.24 ml), and 3 mm and 7.5 mm (resulting in a fluid volume of about 0.15 ml).

The bioreactor 55 is adapted for rotation around a horizontal, rotational axis by associated rotation means (not shown). The base part 56 has a threaded portion 56a in order to facilitate easy and flexible mounting on such rotation means. Typically, the rotational axis is substantially coincident with a central axis through the incubation cavity 55.

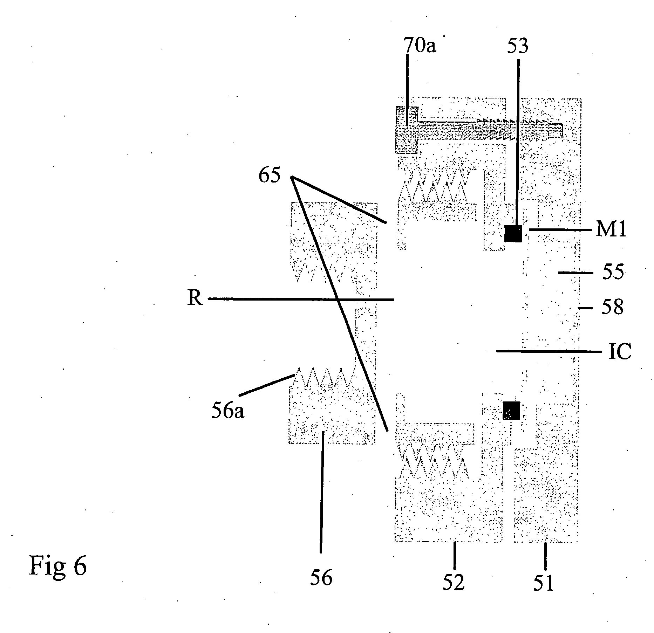

Figure 6 is a schematic drawing of a bioreactor according to the fourth aspect.

Figure 7 shows liquid loss from the incubation chamber of a small volume bioreactor equipped with (lower curve) and without (upper curve) the humidity chamber 60 of the bioreactor 50 according to the second aspect of the invention. In the example, five 1ml bioreactors were filled with DMEM culture medium (without cells) and maintained rotating in a standard, humidified incubator at 37 °C with 5% CO₂. The humidity chamber was filled with pure water. The incubator was in normal use (i.e. the door was opened and closed for the routine cultivation of cells). Loss of water from the incubation chamber was measured in two different ways: firstly as a change in the total amount of water inside the bioreactor (left axis) and secondly in separate experiments as percentage change in the absorption of the indicator phenol red (absorption maximum at 559nM in DMEM, no change in pH of the DMEM was observed, right axis). As shown in Figure 7 (which presents averaged data), there is significant loss of water from the incubation chamber of small volume bioreactors, notwithstanding humidification of the incubator. Without the humidity chamber 60 in front of the incubation cavity 55 there is a significant loss of liquid from the cavity 55 even after 1/2 day, reaching nearly 40% after 5 days. Such a high loss of liquid will concentrate the growth medium, thereby affecting concentration-dependent parameters that can have critical effects on gene expression. The loss of liquid is so rapid that long term growth of cells and/or long-term pharmaceutical or toxicological experiments are rendered impossible to perform without nearly constant manual manipulation (i.e., adding growth medium). In contrast, using the humidity chamber, relative liquid retainment by the incubation cavity 55 is above 95% for 2 days, and above 90% even after 5 days. Accordingly, by practice of the present invention, cell cultures can be maintained in a differentiated state for indefinitely long term periods - cell culture medium can be exchanged every few days and cells occasionally sub-cultured without need for nearly constant efforts to counteract rapid water loss from the incubation chamber.

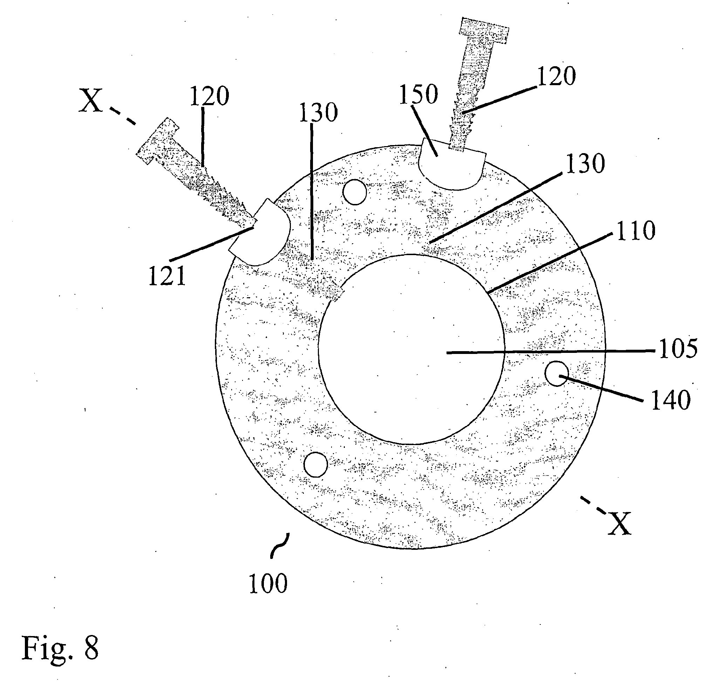

Figure 8 is a cross-sectional drawing of a bioreactor 100 according to the third aspect with an incubation cavity 105 for incubation of cells. The cavity 105 has an inner surface 110. In the embodiment shown in Figure 8, the inner surface 110 has a cylindrical symmetry as viewed from a central axis of the incubation cavity 105. However, application of the present invention is not limited to this geometry but may readily be applied to other shapes as it has recently been observed that improved cultivation may result from spherical, oblate spherical, extended spherical shapes and combinations thereof. See in particular US Patent 6,642,019, which is hereby incorporated by reference in its entirety.

During cultivation of cells the bioreactor 100 will be rotated slowly around a central axis, typically at a rotational frequency of about 5-15 revolutions per minute (RPM). For mounting and clamping of the bioreactor parts the bioreactor comprises a plurality of holes 140, such as three, four or five holes, for mounting on a base member (not shown) whereupon the bioreactor 100 can be mounted and rotated under cultivation.

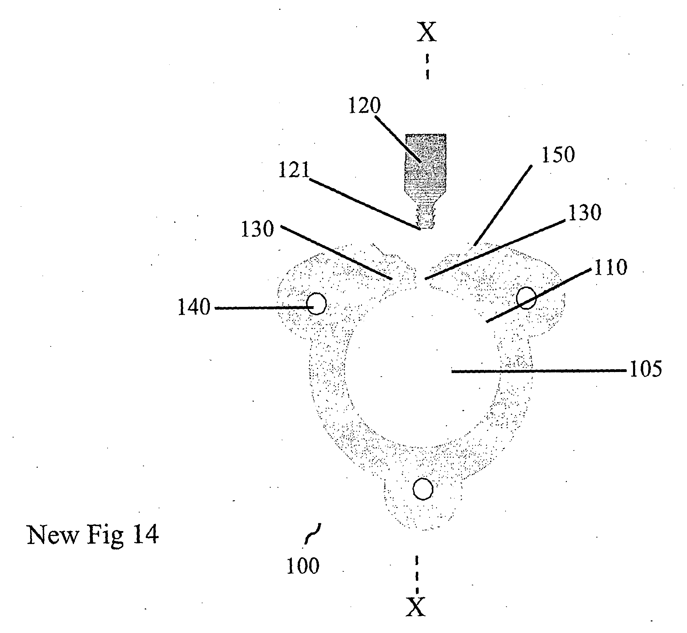

The bioreactor 100 also comprises a plurality of through-going ports 130a and 130b adapted for filling of the incubation cavity 105. In the embodiment shown in Figure 8, the reactor 100 comprises two ports 130a and 130b. The two ports 130a and 130b are arranged azimuthally at different angles as viewed from a central axis of the incubation cavity 105, the first port 130a and the second port 130b being positioned in the shown embodiment with an azimuthal angle difference of about 65 degrees. There is no particular restriction on the azimuthal angle difference, but preferably the angle is less than 90 degrees, more preferably between 70 and 20 degrees and most preferably between 35 and 55 degrees. The skilled person may however readily implement other angles and numbers of ports 130 once the principle of the present invention is recognised. These ports need not be of equal size and one port could be thus larger to provide easier access to the incubation chamber 105 for example for the insertion of tissue biopsies. As a further example, an embodiment of the bioreactor having only one port is illustrated in figure 14.

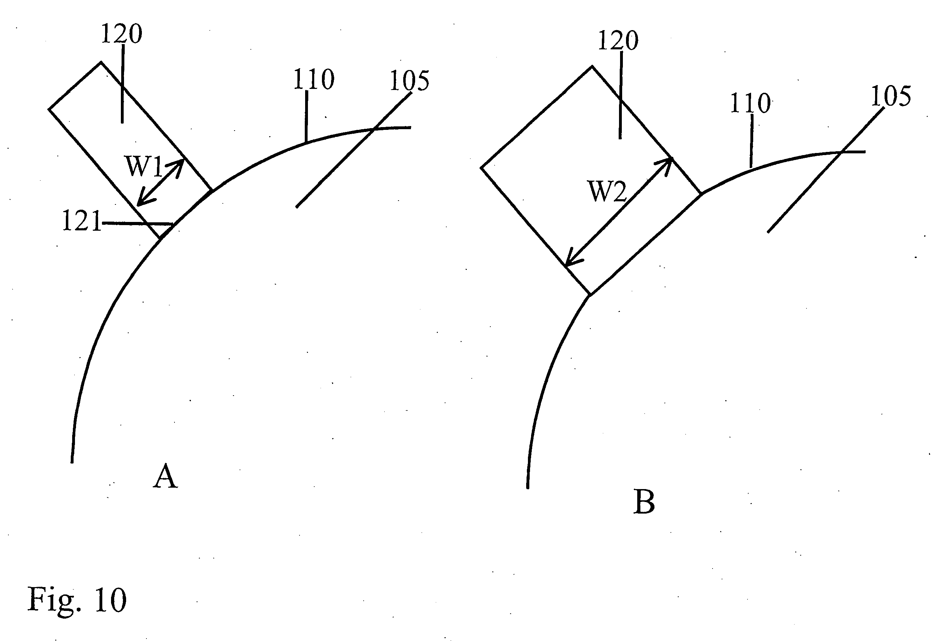

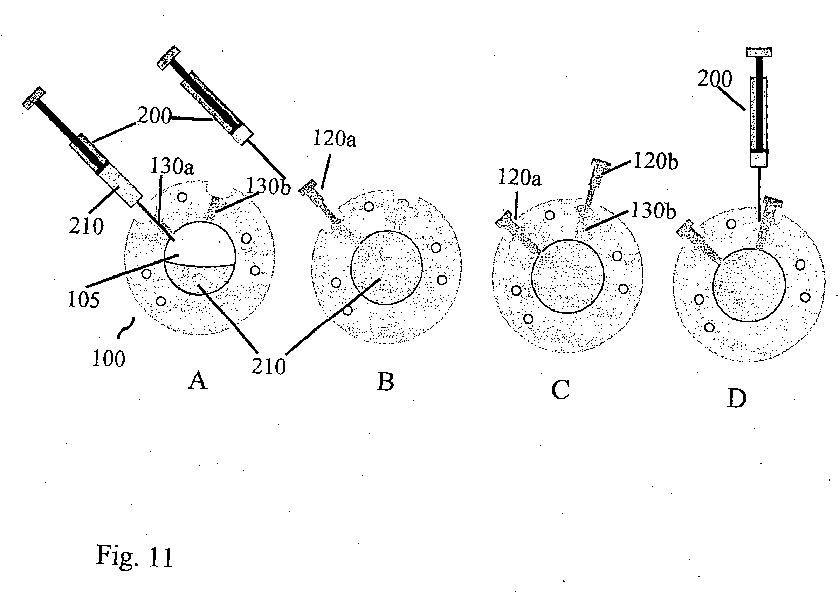

In order to close the ports 130, a plurality of inserts 120 are provided. Each insert 120 is adapted to fit in the corresponding through-going port 130 to provide a substantially fluid tight lock of each port 130 during operation of the bioreactor 100. Inserts 120 may be manufactured in nylon, hard rubber, polyethylene, POM, metals or any other suitable non-cell toxic material for providing a fluid tight closure of the ports 130. Preferably, the inserts 120 has an outer portion being threaded and similarly the ports 130 are adapted for receiving such threaded inserts 120 as indicated in Figure 8. The inserts 120 are further characterized by having an end portion 121 that is substantially aligned with the inner surface 110 of the incubation cavity 105 when inserted in the corresponding port. This will be explained in further details below, see Figure 10. The purpose of the alignment of the end portion 121 and the inner surface 110 is to create an essentially zero volume port, i.e. fluid from the cavity 105 cannot flow into the port 130. Additionally, the alignment of the end portion 121 and the inner surface 110 will minimize or eliminate the turbulence that might otherwise be created in the cavity 105 due to the flow of fluid in and out of the ports 130 and most importantly during its rotation. The ports 130 have outer recessions 150 to facilitate effective filling of the bioreactor 110, eliminating spillage of fluids see Figure 11.

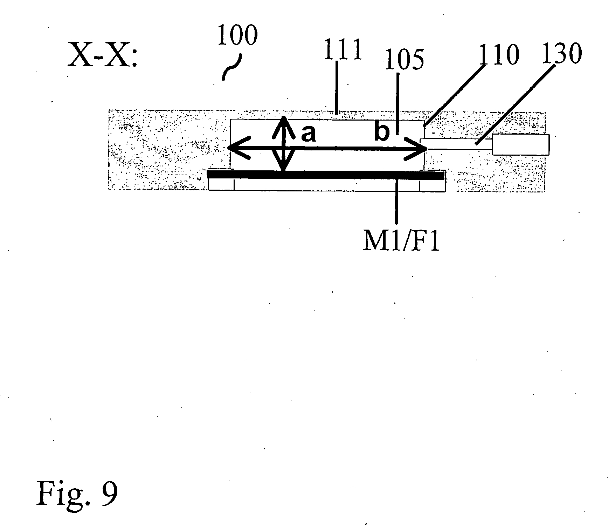

Figure 9 is another cross-sectional drawing of a bioreactor 100 according to the third aspect along the line X-X shown in Figure 8 and 14. In the cross section of Figure 9, only one port 130 is shown. The port 130 is connected to the cavity 105 for easy filling of the reactor 100. In Figure 8, the flexible filter F1 or the flexible membrane M1 is mounted so as to close the rear part of the cavity 105. The bioreactor 100 preferably has a recession in the rear to facilitate easy and fluid tight fastening of the flexible filter F1 or the flexible membrane M1. Typically, additional fastening means (not shown) are provided. If easy access to the incubation cavity is provided via one of the ports 130, then the membrane M1 or filter F1 can be attached (e.g. welded to the bioreactor 100). The function of this flexible part M1/F1 is to almost equalize the overpressure in the cavity 105 that results from overfilling the cavity 105 according to the present invention. Preferred materials for the flexible membrane M1 are polytetrafluroethylene (PTFE), polyvinylidene fluoride (PVDF), silicon rubber, radiation treated plastic or similar materials which are highly water impervious but gas permeable. Preferred materials for the flexible filter F1 are polytetrafluroethylene (PTFE), polyvinylidene fluoride (PVDF), silicon rubber, foam plastics, radiation treated plastic or similar materials which are water and gas permeable.

In the front of the bioreactor 100, a transparent section 111 is located so that the cultivation of cells etc. may be monitored and assessed visually, either manually or automatically, from outside of the bioreactor 100. The transparent section 111 could be made of glass, plastic or any other suitable materials being both transparent and biologically and chemically inert relative to the cultivation process. Preferred materials may include (but are not limited to) various types of glass, polystyrene, polycarbonate, polypropylene, polyethylene and polymethyl methacrylate.

The width "b" of the cavity 105 may be in the range from 4 to 20 mm, such as 6, 8, 10, 12, 14, 16, or 18 mm. The depth "a" of the cavity 105 may be in the range from 2 to 6 mm, such as 2.5, 3, 3.5, 4, 4.5 or 5 mm. Thus, the volume of the cavity 105 is in the range from about 0.03 to 2 ml. Some preferred values of "a" and "b" are 4 mm and 18 mm (resulting in a fluid volume of about 1 ml), 3 mm and 10 mm (resulting in a fluid volume of about 0.24 ml), and 3 mm and 7.5 mm (resulting in a fluid volume of about 0.15 ml). The said dimensions of the inner cavity 105 may be applicable for a bioreactor 10, 50 and/or 100 according to the first, second or third aspect of the invention of any combinations thereof.

Figure 10 is a schematic drawing of a portion of the inner surface 110 of the bioreactor 100 and inserts 120 for closing the cavity 105. Figure 10 illustrates how various widths of the insert 120 can influence the alignment of the inner surface 110 with the end portion 121 of the insert 120. In part A, the width w1 is sufficiently small compared to the radius of curvature of the inner surface section 110 with the result that effectively there is still substantially alignment between the inner surface 110 and the end portion 121. However, in Figure 10 part B the width w2 of the insert 120 is of comparable magnitude to the radius of curvature for the inner surface section 110 and therefore the alignment of the inner surface 110 and the end portion 121 is lower relative to the situation illustrated in part A. Nevertheless, the alignment is still sufficiently high enough for the inner surface 110 and the end portion 121 to be aligned within the context of the present application

In a mathematical approach, a measure for circularity, e.g. radius of curvature or similar, may be introduced and coupled with a fluid dynamic model to assess the turbulence limit so as to avoid tearing of the cell culture or tissue. Thus, a Reynold's number may be estimated to predict the operating conditions for the onset of turbulence with a certain inner surface 110 and end portion 121 configuration. The onset of turbulence is given by a transitional Reynold's number, which is calculated using a characteristic length of the perturbation introduction by the insert 120. For the configuration of the present invention, a transitional Reynold's number may be estimated by use of the diameter of the insert 120 and/or a level difference between the end portion 121 and the inner surface 110.

In a practical approach, the edges of the end portion 121 abutting the inner surface 110 may be flushed or levelled with the inner surface section 110. Irregularities on the edges will induce turbulence and are therefore to be avoided. Hence, the edges of the end portion 121 may be flush to within 3mm, preferably 2 mm and most preferably within 1 mm of the inner surface 110. Note that the insert 120 could be either too short or too long - both options are undesirable.

In order to improve the alignment of the inner surface 110 and the end portion 121 the end portion 121 may itself be designed with a curvature, either inwards or outwards, so as to increase the alignment when inserted in the ports 130. For threaded inserts 120 the curvature should of course be co-ordinated with the threading arrangement so as to ensure correct alignment of the end portion 121 with the inner surface 110 when the inserts 120 are threaded all the way into the fluid locking position.

Figure 11, A to D, is a sequence of schematic drawings illustrating a method for operating a bioreactor 100 according to the third aspect. The bioreactor comprises at least a first 130a and a second 130b through-going port. A syringe or pipette 200 for filling the reactor 100 with liquid 210 is also shown in part A and part B. The syringe 200 in part D is used for removing excess liquid from the outer recess 150 of the ports 130

Initially, the syringe 200 is applied for filling the cavity 105 with liquid 210. The ports 130a and 130b should of course be arranged so that the liquid 210 cannot flow out of the reactor 100 during filling.

As shown in part B, the bioreactor 100 is overfilled with liquid 210 through the first port 130a so that the bioreactor 100 contains liquid 210 at least in the entire incubation cavity 105 and in at least part of the first port 130a and/or in at least part of the second port 130b. This approach for filling the bioreactor may facilitate the removal of effectively all air bubbles from the bioreactor. The corresponding insert 120a is inserted in the first port 130a to lock the first port 130a completely. Thereby all the fluid 210 previously situated in the first port 130a is forced out of the port 130a.

In part C, a corresponding insert 120b is inserting in the second port 130b to lock the second port. Thus, there is provided a modest overpressure (Δp) in the liquid 210 in the incubation cavity 105 but by the functioning of the flexible membrane M1 or flexible filter F1 (not shown) the overpressure will be equalized by an outwards bending of the membrane or the filter away from cavity 105, see Figure 8. Effectively, the cavity 105 has a variable fluid volume. Preferably, the relative increase of the fluid volume in the cavity 105 may be from about 2 to 10%. The overpressure (Δp) may be in the range from 2 to 10 %.

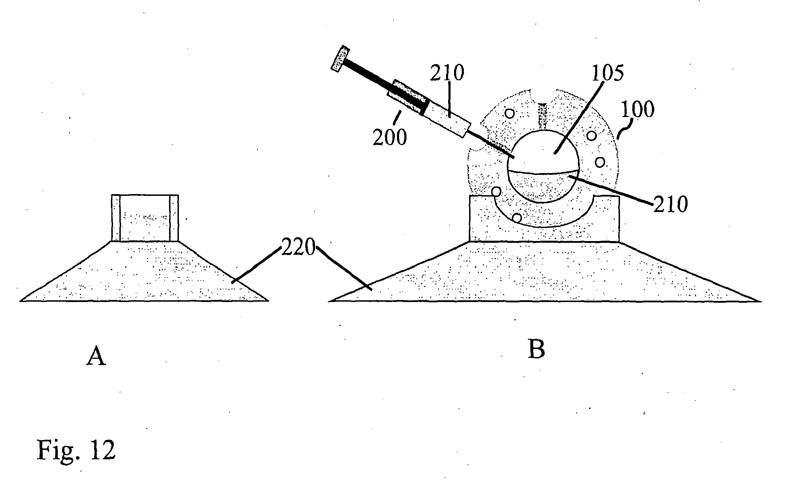

Figure 12 shows a cross-sectional side view (part A) and a cross-sectional front view (part B) of a mount 220 for a bioreactor 100 according to the third aspect. In part B, the bioreactor 100 is resting on the mount 220, the mount 220 and the reactor 100 being arranged so as to allow for relatively small rotations of the bioreactor 100, preferably by manual operation. Thereby it is easier for the user to arrange the bioreactor 100 for filling and/or refilling with liquid, cells, nutrients, removal of air bubbles etc.

A variant of the present invention may include only one inlet port 130 and one corresponding insert 120 (see fig. 14).

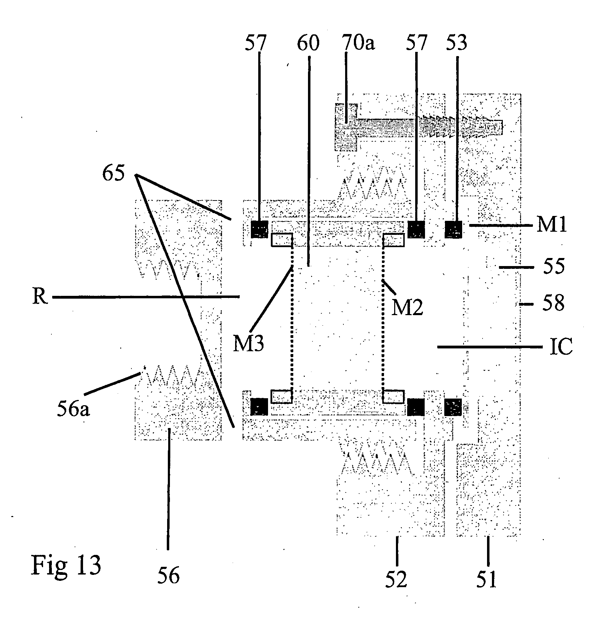

Figure 13 shows an alternative embodiment of a bioreactor shown in Figure 5 according to the second aspect of the invention. Part 52 is adapted so as to be screwed directly into part 56. This modification eliminates 3 screws that were used to hold parts 52 and 56 together and thus simplifies and reduces the hands on time working with the bioreactor. The humidity chamber is held in place using two loosely fitting O rings 57 inside of part 56 and can be rapidly and simply exchanged when needed.

Figure 14 shows an alternative construction of the bioreactor 100 and illustrating a single through going port. Filling this type of construction can be achieved using a syringe with a narrow needle to inject growth medium, forcing the air out of the bioreactor past the needle.

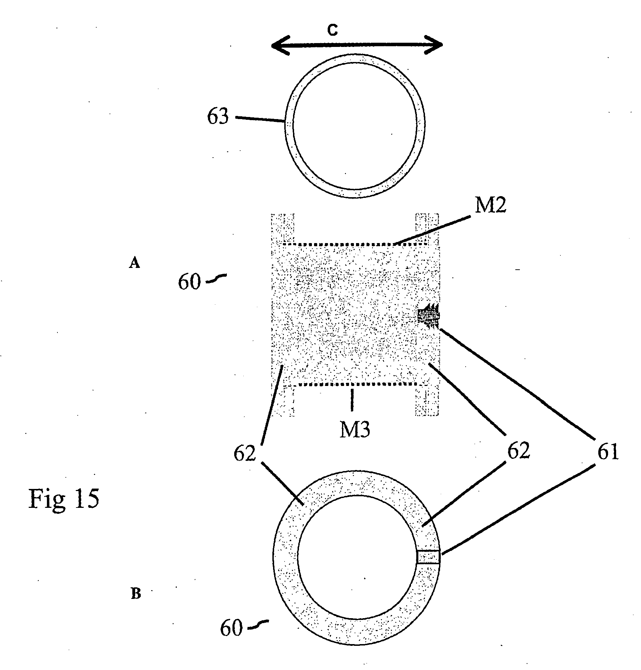

Figure 15 shows an alternative construction of the humidity chamber (shown in figure 4) where the filters have been recessed to protect them from mechanical wear. This design also lends itself to snap closure or ultrasounic welding of the annulus ring 63 to 62 to hold the filters securely in place.

According to a fifth aspect of the present invention, the bioreactor of the invention may be used for the incubation of one or more cell cultures, tissue biopsies, cell clusters, tissue-like structures, "prototissues" or similar samples.

A particular embodiment concerns the use of the bioreactor herein for the incubation of one or more cell cultures, wherein the one or more cell cultures are incubated for an extended period of time, such as 1 week, 2 weeks, or 3 weeks, preferably at least 1 month, such as at least 2, 3, 4, 5, 6, 7, 8, 9, 10, or 11 months, most preferably at least 12 months.

One advantage of using the bioreactor according to the present invention for the incubation of one or more cell cultures is that the cells remain in or achieve a highly differentiated state for an extended period of time. The use of the bioreactor according to the present invention furthermore avoids the use of trypsinisation to obtain the cells from the bioreactor.

According to a sixth aspect of the invention, a method of incubating one or more cell cultures is provided. In one preferred embodiment, the method comprises the steps of:
a) obtaining the one or more cell cultures to be inoculated,
b) transferring the one or more cell cultures to the incubation cavity of a bioreactor according to the invention,
c) filling the incubation cavity with growth medium,
d) optionally eliminating air (bubbles) from the incubation cavity,
e) incubating said cell cultures under microgravity conditions,
f) changing growth medium whenever necessary to sustain incubation for the desired time period, and
g) optionally letting the culture differentiate and form tight junctions or other forms of close cell contacts (e.g. by the secretion of intercellular matrix proteins) between the cells.

In another embodiment, the method comprises the steps of:
a) obtaining the one or more cell cultures to be inoculated,
b) transferring the one or more cell cultures to the incubation cavity of a microgravity bioreactor equipped with means of relative retainment of water in the incubation cavity wherein the incubation cavity has an internal fluid volume of less than 1 ml
c) filling the incubation cavity with growth medium,
d) optionally eliminating air (bubbles) from the incubation cavity,
e) incubating said cell cultures under microgravity conditions,
f) changing growth medium whenever necessary to sustain incubation for the desired time period, and
g) optionally letting the culture differentiate and form tight junctions or other forms of close cell contacts (e.g. by the secretion of intercellular matrix proteins) between the cells,
wherein the relative retainment of water in the incubation cavity is greater than 95% after 2 days or, alternatively, wherein the relative retainment of water in the incubation cavity is greater than 95% after 5 days.

Alternatively, the incubation cavity of the bioreactor used in step b) has an internal fluid volume of less than 900 uL, less than 800 uL, less than 700 uL, less than 600 uL, less than 500 uL, less than 400 uL, less than 300 uL, less than 200 uL, less than 100 uL, less than 50 uL, or 25 uL.

It will be readily understood by one skilled in the art that steps b) and c) could be performed in whole or in part in any order and sequence.

In a particular embodiment, the one or more cell cultures are spheroids or are on microcarrier beads.

In another embodiment, the method of incubating one or more cell cultures comprises replenishing the humidity chamber of the bioreactor of the invention with an aqueous liquid whenever it is necessary to maintain a relative humidity of at least 50%, preferably at least 70%, or even more preferably at least 90%, such as at least 95%, 96%, 97%, 98% or 99%, in the air tight conduit of the bioreactor according to the invention.

In order to reduce the amount of air bubbles in the incubation cavity of the bioreactor according to the invention, it may be an advantage to wet the incubation cavity prior to transferring the cells and growth medium to the incubation cavity. Hence, another embodiment of the method of incubating one or more cell cultures according to the invention further comprises wetting the incubation cavity of the bioreactor according to the invention prior to transferring the cells and growth medium to the incubation cavity.

The cells incubated according to the method of the invention maintain a high level of differentiation. In the case of e.g. hepatocyte cells this means that the cells incubated according to the method of the invention will mimic the behaviour of a real liver. This is an advantage in e.g. toxicology studies, wherein the highly differentiated state of the cells is necessary to achieve a toxicological response matching the response of a real, functioning liver. A further advantage of using small bioreactors according to the present invention for toxicology studies, which often require testing at many different concentrations and at many different time points, is that fewer cells and less growth medium are needed to obtain useful results. This advantage is even more significant when human hepatocytes are used so that the toxicology can be determined in a human tissue microstructure liver piece.

Accordingly, a sixth aspect of the invention concerns a method of creating a molecular profile of the biological effect of a chemical composition, comprising the steps of:
a) contacting an isolated population of one or more cell cultures with a chemical composition; and
b) recording alterations in gene expression or protein expression in the one or more cell cultures in response to the chemical composition to create a molecular profile of the biological effect of the chemical composition;
wherein the one or more cell cultures have been incubated according to the method described above.

The toxicity of a chemical composition may express itself very soon after contacting with one or more cell cultures (acute toxicity). Accordingly, a particular embodiment of the invention concerns a method of creating a molecular profile of the biological effect of a chemical composition as described above, wherein the one or more cell cultures is contacted with the chemical composition for a short period of time, such as at the most seven days, preferably not greater than three days, or even more preferably not greater than one day.

Alternatively, the toxicity of a chemical composition may also express itself after contacting with one or more cell cultures for an extended period of time (chronic toxicity). Therefore, a particular embodiment of the invention concerns a method of creating a molecular profile of the biological effect of a chemical composition as described above, wherein the one or more cell cultures is contacted with the chemical composition for at least one month, preferably at least two months, or even more preferably at least three months.

As mentioned above, it is an advantage to utilize the bioreactor according to the present invention for incubating one or more cell cultures. Similarly, contacting a chemical composition with one or more cell cultures may be carried out by incubating the one or more cell cultures together with the chemical composition. Accordingly, a preferred embodiment of the invention concerns a method of creating a molecular profile of the biological effect of a chemical composition as described above, wherein said contacting is performed by incubating the chemical composition and the one or more cell cultures together according to the method described herein.

The method according to the invention of creating molecular profiles may be repeated for several compounds. In particular, it may be repeated for several chemical compositions of known toxicity thus creating a library of molecular profiles. Therefore, a seventh aspect of the invention concerns a method of compiling a library of molecular profiles of the biological effect of chemical compositions having predetermined toxicities, comprising the steps of:
a) contacting an isolated population of one or more cell cultures with a chemical composition having predetermined toxicities;
b) recording alterations in gene expression or protein expression in the one or more cell cultures in response to the chemical composition to create a molecular profile of the biological effect of the chemical composition; and
c) compiling a library or database of molecular profiles by repeating steps a) and b) with at least two chemical compositions having predetermined toxicities;
wherein the one or more cell cultures have been incubated according to the method described above.

Said method of compiling a library or database of molecular profiles may comprise contacting the one or more cell cultures with a chemical composition for an extended period of time (chronic toxicity) or a short period of time (acute toxicity), such as at least one month, preferably at least two months, or even more preferably at least three months, or not greater than seven days, preferably not greater than three days, or even more preferably not greater than one day. In a preferred embodiment, said contacting is performed by incubating the chemical composition and the one or more cell cultures together according to the method of the invention for incubating one or more cell cultures.

Once a library or database of molecular profiles has been compiled for chemical compositions of predetermined toxicities, said library or database may be compared to the profile of a chemical composition of unknown toxicity. This provides for a method of determining the toxicity of chemical compositions with a reliability that was previously only achieved with samples of adult human tissue, such as liver biopsies. Of course, the availability of such samples of adult human tissue is not very high. Hence, a ninth aspect of the present invention concerns a method of typing toxicity of a test chemical composition, comprising the steps of:
a) creating a molecular profile of the biological effect of the test chemical composition as described in above; and
b) comparing the molecular profile in step a) with a library of molecular profiles of the biological effect of chemical compositions having predetermined toxicities compiled according to the method described above;
wherein the type of toxicity of the test chemical composition is determined by the comparison in step b).

By using the bioreactor of the invention for typing toxicity, the toxicity of a chemical composition of unknown toxicity can be determined with only a small amount of cells, growth medium and chemical composition. Hence, with the method of typing toxicity according to the present invention, it is possible to type the toxicity of a chemical composition with the reliability achieved when using adult human tissue, but at a much lower cost. The method according to the invention for typing toxicity may also be applied in instances where a toxicological profile is normally not created. For instance, a chemical composition of two or more compounds that are each individually non-toxic may turn out to be toxic when administered together (this phenomenon is known as 'drug-drug' interactions (even though there need be no chemical reaction between the two drugs).

Although the present invention has been described in connection with the specified embodiments, it is not intended to be limited to the specific form set forth herein. Rather, the scope of the present invention is limited only by the accompanying claims. In the claims, the term comprising does not exclude the presence of other elements or steps. Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

Each of the references cited herein is hereby incorporated by reference in its entirety.

### EXAMPLES

### Example 1: Maintenance of differentiation state of human liver cells in long-term culture: Expression of cellular proteins

Specific proteins expressed by fully differentiated human liver and not typically found in hepatocytes grown under normal-flat- culture conditions are found in cells cultured using a bioreactor of the present invention. The fully differentiated liver cell proteins can still be found after 302 days in culture.

Human hepatocytes were grown in a bioreactor of the present invention for 302 days and then labelled with [³⁵S]-methionine for 20hrs.

The one or more cell cultures are cultivated as described above under normal culture conditions (e.g. 37°C for human cells), using culture media like Eagles, DMEM or RPMI 1640. Culture media needs to be changed every second or third day depending on the particular cell culture in question. Liver cells require fresh media every second day. The media is changed by stopping the rotation of the bioreactor, waiting for the cells or prototissues to sink towards the bottom and then exchanging approximately 50% of the medium for fresh medium.
Initially, depending on the cell type cells will grow rapidly with a doubling time of 2-3 days but as they differentiate, the doubling time increases so that it can reach weeks or even months. Correspondingly, the frequency with which the cultures need to be subcultivated falls dramatically. Initially subcultivation should be carried out after the first week or two and thereafter at considerably longer intervals (e.g. bimonthly or longer). Subcultivation in these bioreactors is carried out by simply diluting the contents of the bioreactor by a factor of 2 to 4. Trypsin or other enzymes are not used.

Proteins were extracted and analysed by two dimensional gel electrophoresis and mass spectrometry.

The proteins are identified by protein, accession number and protein description in Table 1. The number of isoforms identified relates to the number of gel spots that were positively identified as the protein in question.

**Table 1: Cellular proteins expressed by fully differentiated liver cells found in bioreactor cultures after 302 days in culture and not typically secreted by hepatocytes grown under normal - flat- culture conditions.**

| **Protein** | **Accession No.** | **Protein description (No. of identified isoforms)** |
|---|---|---|
| GTM3 | P21266 | Glutathione S-transferase Mu 3 (1) |
| HS7H | P34931 | Heat shock 70 kDa protein HOM (1) |
| HS71 | P08107 | Heat shock 70 kDa protein 1 (1) |
| HS74 | P34932 | Heat shock 70 kDa protein 4 (5) |
| PDX2 | P32119 | Peroxiredoxin 2 (1) |
| PYC | P11498 | Pyruvate carboxylase (1) |

### Example 2: Maintenance of differentiation state of human liver cells in long-term culture: Viability of secretory mechanisms

Specific proteins secreted by fully differentiated normal human adult liver and not typically secreted by hepatocytes grown under normal - flat- culture conditions are secreted by hepatocytes cultured under microgravity conditions using a bioreactor of the present invention.

Proteins were precipitated from aliquots of growth medium from the tissue cultures of example 1 and analysed by two dimensional gel electrophoresis and mass spectrometry. The proteins are identified by protein, accession number and protein description in Table 2. The number of isoforms identified relates to the number of gel spots that were positively identified as the protein in question.

**Table 2: Proteins secreted by fully differentiated liver cells found in bioreactor culture media and not typically secreted by hepatocytes grown under normal - flat-culture conditions.**

| **Protein** | **Accession No.** | **Protein description (No. of identified forms)** |
|---|---|---|
| ALBU | P02768 | Serum albumin (8) |
| APA1 | P02647 | Apolipoprotein A-I (3) |
| CO3 | P01024 | Complement C3 alpha chain (1) |
| FGL1 | Q08830 | Fibrinogen-like protein 1 (1) |
| FIBB | P02675 | Fibrinogen beta chain (1) |
| MTN3 | 015232 | Matrilin-3 (1) |
| THRB | P00734 | Prothrombin (1) |
| TRFE | P02787 | Serotransferrin (5) |

## Claims

1. A bioreactor adapted for rotation, the reactor comprising
- an incubation cavity, the incubation cavity providing, in conjunction with a first semipermeable filter (F1), a substantially closed confinement, said semipermeable filter (F1) being permeable to molecules up to a predetermined molecular weight or size, allowing an inflow of nutrients and fluid culture medium in the incubation chamber and retainment of cells and cellular aggregates in the incubation cavity,
- an equilibrium chamber, said equilibrium chamber providing a volume for a supply of nutrient factors, the removal of waste products and exchange of gasses like oxygen and carbon dioxide and/or pH controlling substances for one or more cell cultures, tissue biopsies, or cell clusters resident in the incubation cavity, and
- conduction means providing a substantially fluid tight conduit from the semipermeable filter (F1) to the equilibrium chamber,
wherein the internal fluid volume of the incubation cavity is less than 500 µL.

2. A bioreactor according to claim 1, wherein the internal fluid volume of the incubation cavity is selected from the group consisting of:
less than 400 µL, less than 300 µL, less than 200 µL, less than 100 µL, less than 50 µL, and 25 µL.

3. A bioreactor according to claim 1, wherein the incubation cavity has a substantially cylindrical shape.

4. A bioreactor according to claim 3, wherein the bioreactor is adapted for rotation around a horizontal, rotational axis by associated rotation means, said rotational axis being substantially coincident with a central axis through the incubation cavity.

5. A bioreactor according to claim 4, wherein the fluid conduction means has a portion substantially parallel to the rotational axis.

6. A bioreactor according to claim 1 further comprising pumping means for circulating fluid culture medium between the equilibrium chamber and the incubation cavity.

7. A bioreactor according to claim 1, wherein the semipermeable filter is permeable to small molecules, but can prevent bacteria, mycoplasma or other living organisms to get through.

8. A bioreactor according to claim 1, wherein the conduction means has an internal fluid volume selected from the group consisting of:
about 25 µl to about 2 ml, about 50 µl to about 1 ml, and about 0.200 ml.

9. A bioreactor according to claim 1, wherein the equilibrium chamber has an internal fluid volume selected from the group consisting of:
about 25 µl to about 2 ml, about 50 µl to about 1 ml, and about 0.200 ml.

10. A bioreactor according to claim 1, wherein the semipermeable filter has a maximum pore size selected from the group consisting of:
0,2 µm, less than or equal to 100 nm, less than or equal to 50 nm, and less than or equal to 10 nm.

11. A bioreactor adapted for rotation, the bioreactor comprising
- an incubation cavity, the incubation cavity providing, in conjunction with a first semipermeable membrane (M1), a substantially closed confinement
- a humidity chamber comprising an aqueous liquid, the chamber comprising a second semipermeable membrane (M2) and a third semipermeable membrane (M3) both arranged in fluid contact with the aqueous liquid, said third permeable membrane (M3) further being arranged for exchange of gases with an atmosphere surrounding the bioreactor, and
- conduction means providing a substantially air tight conduit from the first semipermeable membrane (M1) to the second semipermeable membrane (M2),
wherein the first, second and third semipermeable membranes (M1, M2, M3) are substantially impermeable to water and substantially permeable to oxygen and carbon dioxide so as to facilitate:
a) aeration of the incubation cavity through the first semipermeable membranes (M1) and the humidity chamber, and
b) substantial retainment of water in the incubation chamber;
wherein the internal fluid volume of the incubation cavity is less than 500 µL.

12. A bioreactor according to claim 11, wherein evaporated water from the humidity chamber and/or the incubation chamber provides a relative humidity in the air tight conduit selected from the group consisting of:
at least 50%, at least 70%, and at least 90%.

13. A bioreactor according to claim 11, wherein the bioreactor, when being operated at 37°C with an aqueous solution or suspension in the incubation cavity, has a relative retainment of water in the incubation cavity after 3 days selected from the group consisting of: at least 80%, at least 90%, and at least 95%.

14. A bioreactor according to claim 11, wherein the bioreactor, when being operated at 37°C with an aqueous solution or suspension in the incubation cavity, has a relative retainment of water in the incubation cavity after 5 days selected from the group consisting of: at least 80%, at least 90%, and at least 95%.

15. A bioreactor according to claim 11, wherein the bioreactor, when being operated at 37°C with an aqueous solution or suspension in the incubation cavity, has a relative retainment of water in the incubation cavity after 10 days selected from the group consisting of: at least 80%, at least 90%, and at least 95%.

## Patentansprüche

1. Zur Rotation angepasster Bioreaktor, wobei der Reaktor folgendes umfasst
- eine Inkubationskavität, wobei die Inkubationskavität in Verbindung mit einem ersten semipermeablen Filter (F1) eine im Wesentlichen geschlossene Begrenzung bereitstellt, wobei der semipermeable Filter (F1) für Moleküle bis zu einem vorher bestimmten Molekulargewicht oder Größe durchlässig ist, wobei ein Zufluss an Nährstoffen und Flüssigkulturmedium in die Inkubationskammer und ein Zurückhalten von Zellen und Zellaggregaten in der Inkubationskammer erlaubt wird.
- eine Äquilibrierungskammer, wobei die Äquilibrierungskammer ein Volumen für eine Versorgung mit Nährstofffaktoren, das Entfernen von Abfallprodukten und einen Austausch von Gasen, wie Sauerstoff oder Kohlendioxid und/oder Substanzen zur pH-Wertkontrolle für eine oder mehrere Zellkulturen, Gewebebiopsien oder Zellcluster, die sich in der Inkubationskavität befinden, bereitstellt, und
- Leitungsmittel, die eine im Wesentlichen flüssigkeitsundurchlässige Rohrleitung von dem semipermeablen Filter (F1) zur Äquilibrierungskammer bereitstellen,
worin das innere Flüssigkeitsvolumen der Inkubationskavität weniger als 500 µl beträgt.

2. Bioreaktor nach Anspruch 1, worin das innere Flüssigkeitsvolumen der Inkubationskavität aus der Gruppe ausgewählt ist, bestehend aus:
weniger als 400 µl, weniger als 300 µl, weniger als 200 µl, weniger als 100 µl, weniger als 50 µl und 25 µl.

3. Bioreaktor nach Anspruch 1, worin die Inkubationskavität eine im Wesentlichen zylindrische Form aufweist.

4. Bioreaktor nach Anspruch 3, worin der Bioreaktor zur Rotation um eine horizontale Rotationsachse durch hinzugefügte Rotationsmittel angepasst ist, wobei die Rotationsachse im Wesentlichen mit einer Zentralachse durch die Inkubationskavität übereinstimmt.

5. Bioreaktor nach Anspruch 4, worin die Leitungsmittel für Flüssigkeiten einen Teil aufweisen, der zur Rotationsachse im Wesentlichen parallel ist.

6. Bioreaktor nach Anspruch 1, der weiterhin Pumpmittel zum Zirkulieren des Flüssigkulturmediums zwischen der Äquilibrierungskammer und der Inkubationskavität umfasst.

7. Bioreaktor nach Anspruch 1, worin der semipermeable Filter für kleine Moleküle durchlässig ist, jedoch verhindern kann, dass Bakterien, Mykoplasma oder andere lebenden Organismen hindurch kommen.

8. Bioreaktor nach Anspruch 1, worin die Leitungsmittel ein inneres Flüssigkeitsvolumen aufweisen, das ausgewählt ist aus der Gruppe, bestehend aus:
ungefähr 25 µl bis ungefähr 2 ml, ungefähr 50 µl bis ungefähr 1 ml und ungefähr 0,200 ml.

9. Bioreaktor nach Anspruch 1, worin die Äquilibrierungskammer ein inneres Flüssigkeitsvolumen aufweist, das ausgewählt ist aus der Gruppe, bestehend aus:
ungefähr 25 µl bis ungefähr 2 ml, ungefähr 50 µl bis ungefähr 1 ml und ungefähr 0,200 ml.

10. Bioreaktor nach Anspruch 1, worin der semipermeable Filter eine maximale Porengröße aufweist, die ausgewählt ist aus der Gruppe, bestehend aus:
0,2 µm, weniger oder gleich 100 nm, weniger oder gleich 50 nm und weniger oder gleich 10 nm.

11. Zur Rotation angepasster Bioreaktor, wobei der Reaktor folgendes umfasst
- eine Inkubationskavität, wobei die Inkubationskavität in Verbindung mit einer ersten semipermeablen Membran (M1) eine im Wesentlichen geschlossene Begrenzung bereitstellt,
- eine Feuchtigkeitskammer, die eine wässrige Flüssigkeit umfasst, wobei die Kammer eine zweite semipermeable Membran (M2) und eine dritte semipermeable Membran (M3) umfasst, die beide mit der Flüssigkeit in Kontakt stehend angeordnet sind, wobei die dritte durchlässige Membran (M3) weiterhin zum Austausch von Gasen mit einer den Bioreaktor umgebenden Atmosphäre angeordnet ist, und
- Leitungsmittel, die eine im Wesentlichen luftundurchlässige Rohrleitung von der ersten semipermeablen Membran (M1) zur zweiten semipermeablen Membran (M2) bereitstellen,
worin die ersten, zweiten und dritten semipermeablen Membranen (M1, M2, M3) im Wesentlichen wasserundurchlässig und im Wesentlichen für Sauerstoff und Kohlendioxid durchlässig sind; vereinfacht dargestellt:
Belüftung der Inkubationskavität durch die ersten semipermeablen Membranen (M1) und die Feuchtigkeitskammer, und
Wasser wird in der Inkubationskammer im Wesentlichen zurückgehalten;
worin das innere Flüssigkeitsvolumen der Inkubationskavität weniger als 500 µl beträgt.

12. Bioreaktor nach Anspruch 11, worin das von der Feuchtigkeitskammer und/oder der Inkubationskammer verdampfte Wasser für eine relative Feuchtigkeit in der luftdichten Rohrleitung sorgt, die aus der Gruppe ausgewählt ist, bestehend aus:
wenigstens 50 %, wenigstens 70 % und wenigstens 90 %.

13. Bioreaktor nach Anspruch 11, worin der Bioreaktor bei Betrieb mit einer wässrigen Lösung oder Suspension in der Inkubationskavität bei 37°C ein relatives Zurückhalten von Wasser in der Inkubationskavität nach 3 Tagen aufweist, das aus der Gruppe ausgewählt ist, bestehend aus: wenigstens 80 %, wenigstens 90 % und wenigstens 95 %.

14. Bioreaktor nach Anspruch 11, worin der Bioreaktor bei Betrieb mit einer wässrigen Lösung oder Suspension in der Inkubationskavität bei 37°C ein relatives Zurückhalten von Wasser in der Inkubationskavität nach 5 Tagen aufweist, das aus der Gruppe ausgewählt ist, bestehend aus: wenigstens 80 %, wenigstens 90 % und wenigstens 95 %.

15. Bioreaktor nach Anspruch 11, worin der Bioreaktor bei Betrieb mit einer wässrigen Lösung oder Suspension in der Inkubationskavität bei 37°C ein relatives Zurückhalten von Wasser in der Inkubationskavität nach 10 Tagen aufweist, das aus der Gruppe ausgewählt ist, bestehend aus: wenigstens 80 %, wenigstens 90 % und wenigstens 95 %.

## Revendications

1. Bioréacteur adapté pour une rotation, le réacteur comportant
- une cavité d'incubation, la cavité d'incubation fournissant conjointement avec un premier filtre semi-perméable (F1) un confinement sensiblement fermé, le filtre semi-perméable (F1) étant perméable aux molécules jusqu'à un poids moléculaire ou une taille prédéterminé permettant un afflux des nutriments et un milieu de cultures de fluides dans la chambre d'incubation et une retenue des cellules et des agrégats cellulaires dans la cavité d'incubation,
- une chambre d'équilibre, la chambre d'équilibre fournissant un volume pour l'alimentation des facteurs nutritifs, l'enlèvement des déchets et l'échange des gaz comme l'oxygène et le dioxyde de carbone et/ou des substances contrôlant le pH pour une ou plusieurs cultures de cellules, des biopsies de tissus ou des groupes de cellules résident dans la cavité d'incubation, et
- des moyens de conduction fournissant un conduit sensiblement étanche aux fluides du filtre semi-perméable (F1) à la chambre d'équilibre,
le volume interne du fluide de la cavité de l'incubation étant inférieur à 500 µL.

2. Bioréacteur selon la revendication 1, le volume interne du fluide de la cavité de l'incubation étant sélectionné du groupe comportant :
inférieur à 400 µL, inférieur à 300 µL, inférieur à 200 µL, inférieur à 100 µL, inférieur à 50 µL et 25 µL.

3. Bioréacteur selon la revendication 1, la cavité de l'incubation présentant une forme sensiblement cylindrique.

4. Bioréacteur selon la revendication 3, le bioréacteur étant adapté pour une rotation autour un axe horizontal de rotation par des moyens de rotation associés, cet axe de rotation étant sensiblement coïncident avec un axe central à travers la cavité d'incubation.

5. Bioréacteur selon la revendication 4, les moyens de conduction de fluide ayant une portion sensiblement parallèle à l'axe de rotation.

6. Bioréacteur selon la revendication 1 comportant en outre des moyens de pompage pour la circulation du milieu de culture de fluide entre la chambre d'équilibre et la cavité d'incubation.

7. Bioréacteur selon la revendication 1, le filtre semi-perméable étant perméable aux petites molécules mais pouvant éviter la pénétration des bactéries, des mycoplasmes ou d'autres organismes vivants.

8. Bioréacteur selon la revendication 1, les moyens de conduction ayant un volume interne du fluide sélectionné du groupe comportant :
environ 25 *l à environ 2ml, environ 50 µl à environ 1 ml, et environ 0,200 ml.

9. Bioréacteur selon la revendication 1, la chambre d'équilibre ayant un volume interne du fluide sélectionné du groupe comportant :
environ 25 µl à environ 2ml, environ 50 µl à environ 1 ml, et environ 0,200 ml.

10. Bioréacteur selon la revendication 1, le filtre semi-perméable ayant une taille de pore maximum sélectionnée du groupe comportant :
0,2 µm, inférieure à ou égale à 100 nm, inférieure à ou égale à 50 nm, et inférieure à ou égale à 10 nm.

11. Bioréacteur adapté pour une rotation, le bioréacteur comportant :
- une cavité d'incubation, la cavité d'incubation fournissant conjointement avec une première membrane semi-perméable (M1) un confinement sensiblement fermé
- une chambre d'humidité comportant un liquide aqueux, la chambre comportant une deuxième membrane semi-perméable (M2) et une troisième membrane semi-perméable (M3) disposées toutes les deux en contact fluide ave le liquide aqueux, la troisième membrane perméable (M3) étant disposée en outre pour l'échange des gaz avec une atmosphère entourant le bioréacteur, et
- des moyens de conduction fournissant un conduit sensiblement étanche à l'air de la première membrane semi-perméable (M1) à la deuxième membrane semi-perméable (M2),
la première, la deuxième et la troisième membrane semi-perméable (M1, M2, M3) étant sensiblement imperméable à l'eau et sensiblement perméable à l'oxygène et au dioxyde de carbone pour faciliter :
a) l'aération de la cavité d'incubation à travers la première membrane semi-perméable (M1) et la chambre d'humidité et
b) une retenue considérable de l'eau dans la chambre d'incubation ;
le volume interne du fluide de la cavité d'incubation étant inférieur à 500 µL.

12. Bioréacteur selon la revendication 11, l'eau évaporée de la chambre d'humidité et/ou de la cavité d'incubation fournissant une humidité relative dans le conduit étanche à l'air sélectionné du groupe comportant :
au moins 50 %, au moins 70 % et au moins 90 %.

13. Bioréacteur selon la revendication 11, le bioréacteur, en opérant à 37°C avec une solution aqueuse ou suspension dans la cavité d'incubation, ayant une retenue relative de l'eau dans la cavité d'incubation après trois jours sélectionnée du groupe comportant : au moins 80 %, au moins 90 % et au moins 95 %.

14. Bioréacteur selon la revendication 11, le bioréacteur, en opérant à 37 °C avec une solution aqueuse ou suspension dans la cavité d'incubation, ayant une retenue relative de l'eau dans la cavité d'incubation après cinq jours sélectionnée du groupe comportant : au moins 80 %, au moins 90 % et au moins 95 %.

15. Bioréacteur selon la revendication 11, le bioréacteur, en opérant à 37 °C avec une solution aqueuse ou suspension dans la cavité d'incubation, ayant une retenue relative de l'eau dans la cavité d'incubation après dix jours sélectionnée du groupe comportant : au moins 80 %, au moins 90 % et au moins 95 %.
